# EUROPEAN PATENT SPECIFICATION

(11) **EP 2 264 462 B1**
(45) Date of publication and mention of the grant of the patent: **07.11.2012**
(21) Application number: 09718200.0
(22) Date of filing: 27.02.2009
(51) Int. Cl.: G01N 33/50, G01N 33/574, C07K 16/18, C07K 16/28

(54) **SCREENING METHOD FOR A THERAPEUTIC AGENT FOR CANCER**
SCREENING-VERFAHREN FÜR EIN THERAPEUTISCHES MITTEL GEGEN KREBS
PROCÉDÉ DE CRIBLAGE D'UN AGENT THÉRAPEUTIQUE POUR LE CANCER

(30) Priority: 04.03.2008 JP 2008053959
(43) Date of publication of application: 22.12.2010
(73) Proprietor: Hisamitsu Pharmaceutical Co., Inc., Tosu-shi, Saga 841-0017 (JP); Chiba-Prefecture, Chiba 260-8667 (JP)
(72) Inventor: NAKAGAWARA, Akira, Chiba-shi Chiba 260-0801 (JP); TAKATORI, Atsushi, Chiba-shi Chiba 260-0801 (JP)
(74) Representative: Grünecker, Kinkeldey, Stockmair & Schwanhäusser
(86) International application number: PCT/JP2009/053699
(87) International publication number: WO 2009/110383

(56) References cited:
- WO-A1-2004/055055
- WO-A2-2009/046123
- JP-T- 2006 525 784
- HAMANO SHIHO ET AL: "Identification of novel human neuronal leucine-rich repeat (hNLRR) family genes and inverse association of expression of Nbla10449/hNLRR-1 and Nbla10677/hNLRR-3 with the prognosis of primary neuroblastomas", INTERNATIONAL JOURNAL OF ONCOLOGY, vol. 24, no. 6, June 2004 (2004-06), pages 1457-1466, XP008133948, ISSN: 1019-6439
- TAKATORI ATSUSHI ET AL: "NLRR family proteins play distinct roles in deciding cell fate and affect the clinical outcome in neuroblastomas", PROCEEDINGS OF THE AMERICAN ASSOCIATION FOR CANCER RESEARCH ANNUAL MEETING, vol. 51, April 2010 (2010-04), page 1275, XP001537394, & 101ST ANNUAL MEETING OF THE AMERICAN-ASSOCIATION-FOR-CANCER-RESEARCH; WASHINGTON, DC, USA; APRIL 17 -21, 2010 ISSN: 0197-016X
- HAMANO ET AL.: 'Shinkei Gashu Subset-kan de Tokuiteki na Hatsugen o Shimesu Shinki Hito NLRR Family Idenshi no Dotei to sono Kaiseki' THE JAPANESE CANCER ASSOCIATION SOKAI KIJI, 61ST (2002) page 259
- HOSSAIN MS ET AL.: 'N-MYC promotes cell proliferation through a direct transactivation of neuronal leucine-rich repeat protein-1 (NLRR1) gene in neuroblastoma' ONCOGENE vol. 27, 30 June 2008, pages 6075 - 6082

## Description

### Technical Field

The present invention relates to a screening method for a therapeutic agent for cancer. More particularly, the present invention relates to a screening method for a therapeutic agent for cancer, which uses the interaction between NLRR1 (neuronal leucine-rich repeat protein 1; SEQ ID NO:2) and EGFR (epidermal growth factor receptor) as an indicator.

### Background Art

NLRR family genes are expressed in the relatively normal nervous system, and, from the analysis of the expression in the development process of mouse, are thought to play an important role in processes for regulating proliferation, differentiation and cell death of cells in the formation of the nervous system and organs in fetal life (Non Patent Literature 1).

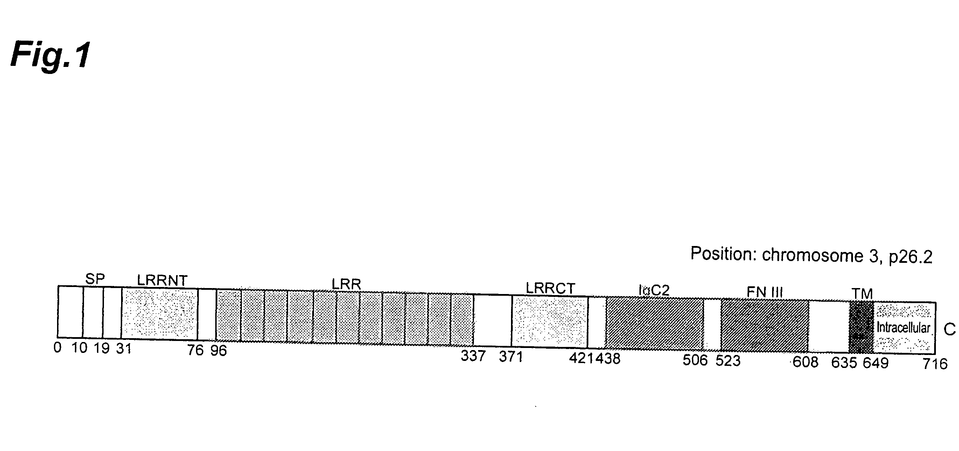

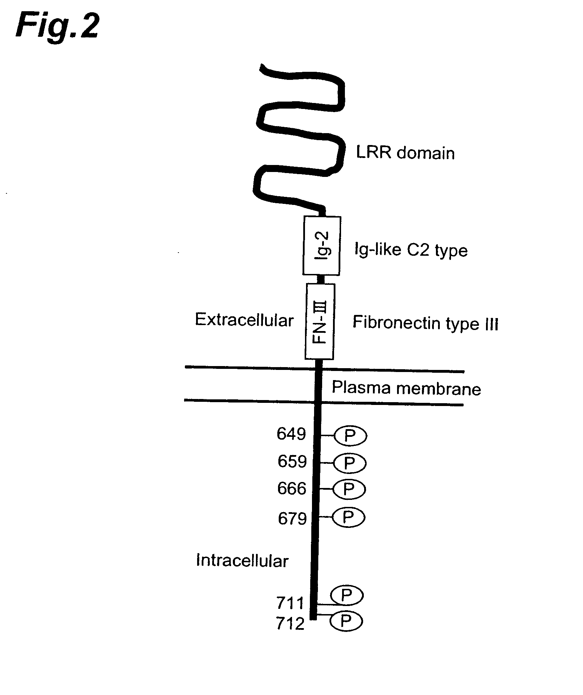

The present inventors have isolated about 5400 genes from neuroblastoma cDNA library (Patent Literature 1). The present inventors have identified human NLRR1 from these genes as a gene whose expression amount is different between neuroblastoma with a good prognosis and neuroblastoma with a poor prognosis (Non Patent Literature 2). Figure 1 and Figure 2 are views showing a structure of NLRR1. As shown in Figure 1, NLRR1 is a single transmembrane protein having 11 leucine-rich repeats. Furthermore, as shown in Figure 2, the intracellular domain of NLRR1 possesses a tyrosine residue that can be phosphorylated.

The present inventors have clarified that the expression of NLRR1 is significantly high in neuroblastoma that has a poor prognosis and is refractory (Non Patent Literature 2). Furthermore, the present inventors have found that transcription of NLRR1 is directly induced by Myc (N-Myc and c-Myc transcription factors) that is a typical oncogene. Thus, the relation between NLRR1 and cancer has been strongly suggested, but the function of NLRR1 has not been clear.

### Citation List

### Patent Literature

Patent Literature 1: WO 01/66719

### Non Patent Literature

Non Patent Literature 1: Haines BP, et al., Developmental Biology, 281,145-159 (2005)

Non Patent Literature 2: Hamano S, et al., Int. J. Oncol. 24(6), 1457-66 (2004)

### Summary of Invention

### Technical Problem

Clarification of the function of NLRR1 leads to the elucidation of the relation between NLRR1 and cancer, and, to the development of a therapeutic agent for cancer based on a new mechanism. Therefore, one object of the present invention is to clarify the function of NLRR1. Another object of the present invention is to provide a screening method for a therapeutic agent for cancer based on a new mechanism from the elucidated findings.

### Solution to Problem

As shown below, the present inventors have clarified in the present invention that NLRR1 and EGFR interact with each other to form a complex. Furthermore, the present inventors have clarified that this interaction remarkably increases the cell proliferation signal from the EGFR. Furthermore, the present inventors have clarified that the NLRR1 is involved in the malignant alteration of a variety of cancer types including refractory neuroblastoma, especially, many refractory cancers in which amplification or expression increase of N-Myc or c-Myc was found. Therefore, a substance that suppresses the interaction between NLRR1 and EGFR could be a therapeutic agent for cancer.

The present invention provides a screening method for a therapeutic agent for cancer, which includes a step of measuring a binding of NLRR1 and EGFR under each condition of being in the presence of a test substance and in the absence of a test substance; and a step of determining that the test substance is a therapeutic agent for cancer when the binding between NLRR1 and EGFR in the presence of the test substance is weaker than the binding between NLRR1 and EGFR in the absence of the test substance wherein the NLRR1 consists of the amino acid sequence as set forth in SEQ ID NO:2. The screening method is based on a molecular mechanism in which NLRR1 and EGFR directly interact with each other to form a complex and to increase an intracellular proliferation signal from EGFR remarkably. This molecular mechanism has been found by the present inventors in the present invention, which provides a screening method for a therapeutic agent for cancer based on a new mechanism.

The present invention also provides a screening method for a therapeutic agent for cancer, which includes a step of culturing cells expressing NLRR1 and EGFR under each condition of being in the presence of a test substance and in the absence of a test substance; a step of measuring a binding of NLRR1 and EGFR in each cultured cells; and a step of determining that the test substance is a therapeutic agent for cancer when the binding between NLRR1 and EGFR in the cells cultured in the presence of the test substance is weaker than the binding between NLRR1 and EGFR in the cells cultured in the absence of the test substance wherein the NLRR1 consists of the amino acid sequence as set forth in SEQ ID NO:2. According to this screening method, screening of a therapeutic agent for cancer can be carried out based on a new mechanism.

The present invention provides a kit for measuring binding of NLRR1 and EGFR, which includes an antibody recognizing NLRR1 and an antibody recognizing EGFR wherein the NLRR1 consists of the amino acid sequence as set forth in SEQ ID NO:2. The use of such a kit makes it possible to carry out a step of measuring the interaction between NLRR1 and EGFR in the above-mentioned screening method in a simple and easy manner.

### Advantageous Effects of Invention

According to the present invention, a screening method for a therapeutic agent for cancer based on a new mechanism is provided.

### Brief Description of Drawings

Figure 1 is a view showing a structure of NLRR1. SP: signal peptide, LRR: leucine-rich repeat, IgC2: immunoglobulin-like C2, FNIII: fibronectin type III, TM: transmembrane;
Figure 2 is a view showing a structure of NLRR1, showing the position of tyrosine residue that can be phosphorylated. LRR: leucine-rich repeat, Ig: immunoglobulin;
Figure 3 is a photograph of an immunoblot showing a binding of EGFR and NLRR1. NLRR1-HA: NLRR1 tagged with a HA tag, IP-EGFR: immunoprecipitation using an anti-EGFR antibody, IB-HA: an immunoblot using an anti-HA antibody, IB-EGFR: an immunoblot using an anti-EGFR antibody;
Figure 4 is a graph showing a binding of EGF to cells;
Figure 5 is a photograph showing phosphorylation of ERK and Akt. NLRR1-HA: NLRR1 tagged with a HA tag, IGF: insulin-like growth factor, p-ERK: phosphorylated ERK, and p-Akt: phosphorylated Akt;
Figure 6 is a photograph of an immunoblot showing a sugar chain modification of NLRR1 protein;
Figure 7 is a photograph of an immunoblot showing a phosphorylation of a tyrosine residue of NLRR1. IP: immunoprecipitation, IB: immunoblot. NLRR1.myc: NLRR1 tagged with a myc tag;
Figure 8 is a photograph of an immunoblot showing a phosphorylation of a tyrosine residue of NLRR1. Anti-myc antibody was used for IP. FBS: fetal bovine serum;
Figure 9 is a graph showing a proliferation of cells. OD: optical density;
Figure 10 is a photograph of an immunoblot showing a phosphorylation of p38MAPK. NLRR1.myc:NLRR1 tagged with a myc tag;
Figure 11 is a graph showing a proliferation of cells. si-NLRR1: siRNA against NLRR1, OD: optical density;
Figure 12 is a photograph of an immunoblot showing an expression of NLRR1;
Figure 13A is a photograph showing a result of an examination of an mRNA expression of NLRR1 and GAPDH by RT-PCR; and Figure 13B is a photograph of an immunoblot showing an expression of NLRR1.NLRR1.myc: NLRR 1 tagged with a myc tag;
Figure 14 is a graph showing a proliferation of control cells and a cell line continuously expressing NLRR1. NLRR1.myc: NLRR1 tagged with a myc tag;
Figure 15 is a graph showing a proliferation of cells. siNLRR1: siRNA against NLRR1, OD: optical density;
Figure 16 is a photograph showing a result of an immunoblot;
Figure 17 shows results of flow cytometry showing a proportion of sub-G1 cells;
Figure 18 is a graph showing a proportion of sub-G1cells. siNLRR1: siRNA against NLRR1;
Figure 19 is a photograph showing a result of an immunoblot;
Figure 20 is a photograph of an immunoblot in which an expression of MYCN is analyzed;
Figure 21 is a photograph of an immunoblot in which an expression of an extracellular domain of NLRR1 is detected;
Figure 22 is a photograph of an immunoblot in which an expression of an intracellular domain of NLRR1 is detected;
Figure 23 is a graph showing a proliferation of cells. OD: optical density;
Figure 24 is a photograph showing a result of an immunoblot. Myc-NLRR1: NLRR1 tagged with a myc tag, p-ERK: phosphorylated ERK;
Figure 25 is a photograph showing a result of an immunoblot. NLRR1-HA: NLRR1 tagged with a HA tag, p-Akt: phosphorylated Akt;
Figure 26 is a graph showing a proliferation of cells. OD: optical density;
Figure 27 is a photograph showing a result of an immunoblot. FTI: FTI277;
Figure 28 is a graph showing a proliferation of cells. SKNBE: SK-N-BE cell, siNLRR1: siRNA against NLRR1, SY5Y: SH-SY5Y cell, SAN: SMS-SAN cell;
Figure 29 is a photograph showing a result of an immunoblot. P-ERK: phosphorylated ERK, P-p38MAPK: phosphorylated p38MAPK;
Figure 30 is a photograph showing a result of an immunoblot. W.T.NLRR1.myc: wild type NLRR1 tagged with a myc tag, NLRR1.myc:NLRR1 tagged with a myc tag, P-ERK1/2: phosphorylated ERK1/2, P-p38MAPK: phosphorylated p38MAPK, P-p90RSK: phosphorylated p90RSK;
Figure 31 is a photograph showing a result of an immunoblot. FBS: fetal bovine serum, NLRR1.myc: NLRR1 tagged with a myc tag, P-ERK1/2: phosphorylated ERK1/2, ΔN NLRR1: NLRR1 from which an extracellular domain is deleted;
Figure 32 is a photograph showing a result of an immunoblot. FBS: fetal bovine serum, NLRR1.myc: NLRR1 tagged with a myc tag, P-ERK1/2: phosphorylated ERK1/2, ΔC NLRR1: NLRR1 from which an intracellular domain is deleted;
Figure 33 is a photograph showing a result of an immunoblot. W.T.NLRR1.myc: wild type NLRR1 tagged with a myc tag;
Figure 34 is a photograph showing a result of an immunoblot. NLRR1.myc: NLRR1 tagged with a myc tag;
Figure 35 is a photograph showing a result of an immunoblot. ΔC NLRR1.myc: NLRR1 tagged with a myc tag from which an intracellular domain is deleted;
Figure 36 is a photograph showing a result of an immunoblot. ΔN NLRR1.myc: NLRR1 tagged with a myc tag from which an extracellular domain is deleted;
Figure 37 is a photograph showing a result of an immunoblot. P-ERK1/2: phosphorylated ERK1/2;
Figure 38 is a view showing a control of NLRR1 by MYCN; and
Figure 39 is a photograph showing a result of an immunoblot. mβCD: methyl-β-cyclodextrin, NLRR1-HA: NLRR1 tagged with a HA tag, p-EGFR: phosphorylated EGFR, p-ERK: phosphorylated ERK.

### Description of Embodiments

### (Screening Method for Therapeutic Agent for Cancer)

A screening method for a therapeutic agent for cancer of the present invention includes a step of measuring a binding between NLRR1 and EGFR under each condition of being in the presence of a test substance and in the absence of a test substance; and a step of determining that the test substance is a therapeutic agent for cancer when the binding between NLRR1 and EGFR in the presence of the test substance is weaker than the binding between NLRR1 and EGFR in the absence of the test substance. The interaction between NLRR1 and EGFR specifically denotes binding therebetween, that is, a formation of a complex. For the measurement of the interaction, measurement systems for measuring the interaction between proteins, which are well known to a person skilled in the art, can be used, and examples of the measurement systems include a yeast two-hybrid method, a PCA (protein fragment complementation assay) method, an immunoprecipitation method, an analysis by BIAcore (trade name), a gel shift method, and the like. The yeast two-hybrid method and the PCA method are briefly described below.

Firstly, the yeast two-hybrid method is described. Yeast Gal4 is a transcriptional regulating factor composed of an N-terminal DNA-binding domain (DBD) and a C-terminal transcriptional activation domain (AD). Basically, both domains autonomously function and DBD can bind to DNA by itself but cannot activate transcription. AD is the converse. The yeast two-hybrid method was developed by applying this property. That is to say, when a fusion protein (bait) of the protein P of interest with DBD of Gal4 and a fusion protein (prey) of another protein Q with AD of Gal4 are introduced into yeast cells, if the proteins P and Q interact with each other in the nucleus, a transcriptional regulation complex is reconstituted in the yeast cell, resulting in transcriptional activation dependent on a binding site for Gal4. By using reporter genes, the interaction between the proteins P and Q can be evaluated easily by detecting this activity. Examples of the reporter genes can include HIS3, lacZ, and URA3. In addition to yeast Gal4, a system using SRF or LexA is also available.

The screening method of the present invention may be carried out by a system using NLRR1 as the protein P and EGFR as the protein Q (the converse is possible) in the yeast two-hybrid method. That is to say, yeasts are allowed to express any one of NLRR1 and EGFR as a bait and the other of NLRR1 and EGFR as a prey, and the yeasts are cultured under each condition of being in the presence of a test substance and in the absence of a test substance; transcriptional activities (which represent the intensity of interaction between NLRR1 and EGFR) of reporter genes of the cultured yeasts are measured; and when the transcription activity in the presence of the test substance is decreased from the transcription activity in the absence of the test substance, it can be determined that the test substance is a therapeutic agent for cancer.

Next, the PCA method is described. In the PCA method, one functional protein A (for example, enzyme, transcriptional factor) is divided into two fragments A1 and A2, which are then fused to the proteins P and Q of interest, respectively, to prepare fusion proteins A1-P and A2-Q. This method is based on the principle in which if the proteins P and Q of interest bind to each other, the functional protein A recovers its function, and, by detecting the activity thereof, the interaction between the proteins P and Q is determined. Examples of the functional protein may include β-lactamase. Hereinafter, the PCA method using β-lactamase is described.

The β-lactamase is a β-lactam ring-cleaving enzyme derived from bacteria. The β-lactamase is divided into the N-terminal α197 fragment (25 to 197 residues) and the C-terminal ω198 fragment (198 to 288 residues), which are respectively expressed as fusion proteins with proteins of interest. Only when they are bound to each other, the β-lactamase protein recovers its three-dimensional structure and exhibits the activity. The β-lactamase activity is detected with a cell-permeable fluorescent probe CCF2/AM (CCF2/acetoxymethyl ester). The CCF2/AM has a structure in which two different fluorescent substances, coumarin and fluorescein, are bound to both ends of a cephalosporin molecule, and exhibits intramolecular FRET (fluorescence resonance energy transfer) using coumarin as a donor and fluorescein as an acceptor. That is to say, the excitation of coumarin with light of 409 nm results in the emission of fluorescence derived from fluorescein at 520 nm. However, if CCF2 is degraded by the β-lactamase activity, since the two fluorescent substances dissociate from each other, and FRET is not observed, so that coumarin emits its original fluorescence at 447 nm by the excitation with light of 409 nm. The measurement of the fluorescence intensity at 447 nm allows the measurement of the β-lactamase activity, that is, the intensity of interaction between the proteins of interest.

Thus, the screening method of the present invention using the PCA method may be carried out by the following procedures. Cells are allowed to express fusion proteins of two functional protein fragments A1 and A2 fused with NLRR1 on one side, and EGFR on the other side, and cultured under each condition of being in the presence of a test substance or in the absence of a test substance; the respective functional protein activities in the cultured cells are measured; and it can be determined that the test substance is a therapeutic agent for cancer when the activity of the functional protein in the cells cultured in the presence of the test substance is lower than the activity of the functional protein in the cells cultured in the absence of the test substance.

Furthermore, the screening method for a therapeutic agent for cancer of the present invention includes a step of culturing cells expressing NLRR1 and EGFR under each condition of being in the presence of a test substance and in the absence of a test substance; a step of measuring a binding between NLRR1 and EGFR in each cultured cells; and a step of determining that the test substance is a therapeutic agent for cancer when the binding between NLRR1 and EGFR in the cells cultured in the presence of the test substance is weaker than the binding between NLRR1 and EGFR in the cells cultured in the absence of the test substance. The measurement of the interaction can be carried out by using systems for measuring the interaction between proteins, which are well known to a person skilled in the art, and, for example, an immunoprecipitation method can be used. Hereinafter, the screening method using the immunoprecipitation method is described.

Firstly, cells expressing NLRR1 and EGFR are prepared. The cells can be prepared by introducing an expression plasmid of NLRR1 and EGFR into, for example, 293 cells. For facilitating the analysis, NLRR1 and EGFR genes to be introduced may be linked to genes encoding labeling peptide such as a HA tag, a FLAG tag, and a Myc tag. Then, cells expressing NLRR1 and EGFR are cultured under each condition of being in the presence of a test substance and in the absence of a test substance.

Next, the interactions between NLRR1 and EGFR in each cultured cells are measured. To measure the interaction, the cultured cells are firstly homogenized to prepare cell lysates. Before homogenization of cells, EGF may be added to the medium so as to stimulate EGFR. The stimulation is preferably carried out by adding, for example, EGF at the concentration of 10 ng/ml to the medium and culturing for three minutes. Furthermore, before cells are homogenized, a protein cross-linker such as DSP (dithiobis[succinimidyl propionate]) may be reacted. Although cell lysates from the whole cells may be used, it is preferred that cell lysates from the cell membrane fractions are used because the interaction between NLRR1 and EGFR occurs on the cell membrane. Immunoprecipitation is carried out by adding an antibody against either of NLRR1 or EGFR to the prepared cell lysates. Alternatively, immunoprecipitation may be carried out by using an antibody against labeling peptide linked to these proteins. Then, the obtained precipitate (containing a complex of NLRR1 and EGFR) is subjected to an immunological approach (for example, immunoblot) using an antibody against the other molecule, by which the interaction between NLRR1 and EGFR can be measured by detecting and quantifying the complex of NLRR1 and EGFR.

As a result of the measurement, when the interaction between NLRR1 and EGFR in the cells cultured in the presence of the test substance is weaker than the interaction between NLRR1 and EGFR in the cells cultured in the absence of the test substance (when the formation amount of protein complex is smaller), it can be determined that the test substance is a therapeutic agent for cancer.

Examples of the above-mentioned test substances may include a low molecular weight compound, an antibody, an antibody fragment, an antibody derivative, lectin and aptamer. As a supply source of the low molecular weight compound, for example, a commercially available compound library can be used. The preferable test substance can further include an antibody, an antibody fragment, an antibody derivative and aptamer whose antigen is NLRR1 and EGFR, as well as lecithin, and the like. These test substances are easily available, and techniques for mass production of therapeutic agents for cancer have been established.

As described above, the expression of NLRR1 is significantly high in neuroblastoma that has a poor prognosis and is refractory. Furthermore, as mentioned below, enhancement of a proliferation signal by NLRR1 is found not only in an EGFR but also in an IGF (insulin-like growth factor) receptor (IGFR) that is the other membrane receptor for sending a proliferation signal. Therefore, it is thought that NLRR1 is involved not only in refractory neuroblastoma but also in various cancers. Furthermore, as mentioned above, NLRR1 is directly induced to transcription by Myc (N-Myc and c-Myc transcription factors) that is a typical oncogene. Thus, it is thought that NLRR1 is deeply involved in the malignant alteration of many refractory cancers in which amplification or expression increase of N-Myc and c-Myc is observed.

Therefore, a therapeutic agent for cancer obtained by a screening method of the present invention may be applied to many refractory cancers in which amplification or increased expression of N-Myc or c-Myc is observed, more preferably, may be applied to neuroblastoma, and further preferably, may be applied to neuroblastoma that has a poor prognosis and is refractory.

Furthermore, a screening method of the present invention may further include a step of measuring a proliferation rate of cells expressing NLRR1 and EGFR under each condition of being in the presence of a test substance and in the absence of a test substance.

A therapeutic agent for cancer obtained by a screening method of the present invention may be used as a lead compound, and subjected to chemical modification, and thereby it may be possible to obtain pharmaceutical preparation having high activity and improved physical property, pharmacokinetics, and toxicity.

The present invention further provides a kit for measuring a binding of NLRR1 and EGFR, which includes an antibody recognizing NLRR1 and an antibody recognizing EGFR. The use of such a kit allows a step of measuring the interaction between NLRR1 and EGFR in the above-mentioned screening method to be carried out in an easy and simple manner by, for example, an immunoprecipitation method. These antibodies may be prepared by immunizing animals such as a mouse, a rabbit, or the like, with, for example, entire or a part of NLRR1 and EGFR protein as an antigen, or may be selected from the phage display library of antibodies.

The above-mentioned kit may further include expression plasmids of NLRR1 and EGFR. These expression plasmids are inserted into cultured cells such as 293 cells, and thereby cells expressing NLRR1 and EGFR can be obtained. The cells can be used for the above-mentioned screening method for a therapeutic agent for cancer. Furthermore, the above-mentioned expression plasmids of NLRR1 and EGFR may have a structure in which NLRR1 and EGFR proteins are expressed as fused proteins with a labeled peptide such as a myc tag or a HA tag. In this case, an antibody included in the kit may recognize a labeled peptide linked to NLRR1 and EGFR instead of recognizing NLRR1 and EGFR.

The above-mentioned kit may further include a cell line that highly expresses NLRR1 and EGFR. This cell can be used for the above-mentioned screening method for a therapeutic agent for cancer.

### Example

Hereinafter, the present invention is described more specifically with reference to Examples of the present invention. However, the present invention is not intended to be limited to these Examples, and a variety of modifications can be carried out within the scopes of the claims.

### (Materials)

MTT assay kit (Cell counting kit) was purchased from Wako Pure Chemical Industries, Ltd. In vitro Transcription/Translation System (L1170) was purchased from Promega. pCDNA3.1 was purchased from Invitrogen. U0126 and LY294002 were purchased from Cell Signaling Technology. AG1478, FTI277, SP600125 and SB203580 were purchased from Calbiochem. EGF (epidermal growth factor), G418 and Tunicamycine were purchased from Sigma. An isotope-labeled ¹²⁵I-EGF was purchased from GE Healthcare. IGF was purchased from Sigma. DAPI (4'6-diamino-2-phenylindole) and PI (propidium iodide) were purchased from Molecular Probes. DSP was purchased from PIERCE. An anti-myc antibody (9B11), an anti-phosphorylated p38MAPK antibody, an anti-p38MAPK antibody, an anti-phosphorylated-EGFR antibody, an anti-EGFR antibody, an anti-phosphorylated-ERK antibody, an anti-ERK antibody, an anti-phosphorylated-Akt antibody, an anti-Akt antibody, and an anti-phosphorylated p90RSK antibody were purchased from Cell Signaling Technology. An anti-phosphorylated tyrosine antibody (PY20) was purchased from BD Science. An anti-actin antibody and an anti-HA antibody were purchased from Sigma. An anti-MYCN antibody was purchased from Oncogene Research Products.

### (Example 1)

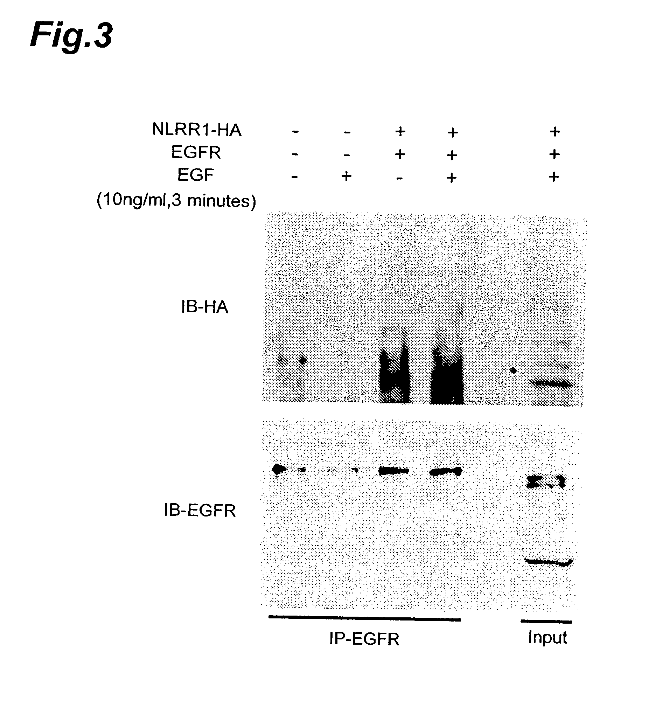

An expression plasmid encoding EGFR and an expression plasmid encoding NLRR1 tagged with an HA tag were introduced into 293 cells to force the expression. The cells were cultured for three minutes in a medium containing 10 ng/ml EGF. The cells were washed with phosphate-buffered saline (PBS), then subjected to cross-linking with DSP, and the cells were collected and subjected to immunoprecipitation with an anti-EGFR antibody. Next, samples were subjected to SDS-polyacrylamide gel electrophoresis, and transferred to a membrane, followed by an analysis of binding between EGFR and NLRR1 by an immunoblot method using an anti-HA antibody and an anti-EGFR antibody. Figure 3 is a photograph showing a result of an immunoblot. As a result, it has been clarified that by the co-expression of EGFR and NLRR1, binding between EGFR and NLRR1 is observed and the binding is enhanced by EGF treatment.

### (Example 2)

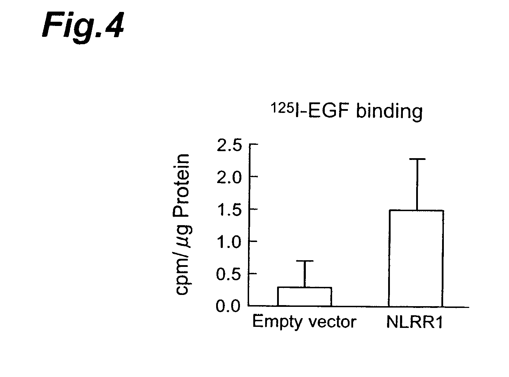

An expression plasmid encoding NLRR1 was introduced into MCF-7 cells to force the expression. As a control, cells into which an empty vector plasmid was introduced were used. These cells were cultured in a medium containing 1 nM isotope-labeled ¹²⁵I-EGF on ice for 60 minutes. The cells were washed with PBS that had been cooled to 4°C, and the cells were collected by using 1M NaOH, followed by measuring the radioactivity detected from the collected cells with the use of a gamma-ray measuring device. The measurement result was corrected by the amount of protein contained in a sample. Figure 4 is a graph showing an experiment result. As a result, it has been clarified that the expression of NLRR1 promotes binding of EGF to cells.

### (Example 3)

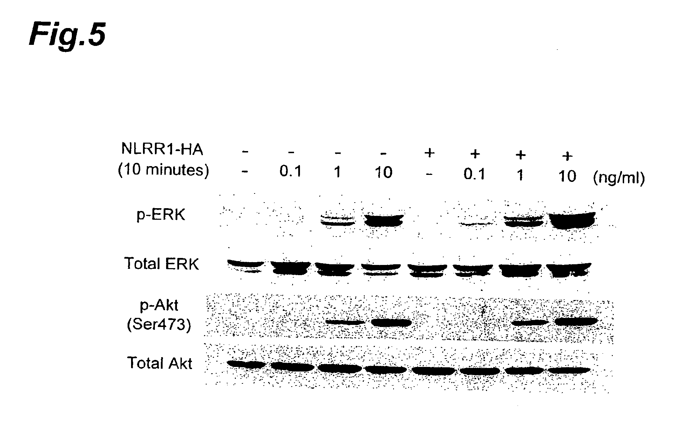

An expression plasmid encoding NLRR1 tagged with a HA tag was introduced into MCF-7 cells to force the expression. As a control, cells into which an empty vector plasmid was introduced were used. These cells were cultured in media containing IGF at the concentrations of 0, 0.1, 1, and 10 ng/ml, and cells were collected after 10 minutes. These cells were subjected to immunoblot using an anti-phosphorylated-ERK antibody, an anti-ERK antibody, an anti-phosphorylated-Akt antibody and an anti-Akt antibody. Figure 5 is a photograph showing a result of an immunoblot. As a result, it has been shown that by the forced expression of NLRR1, phosphorylation of ERK and Akt by treatment with IGF is enhanced as compared with the control.

### (Example 4)

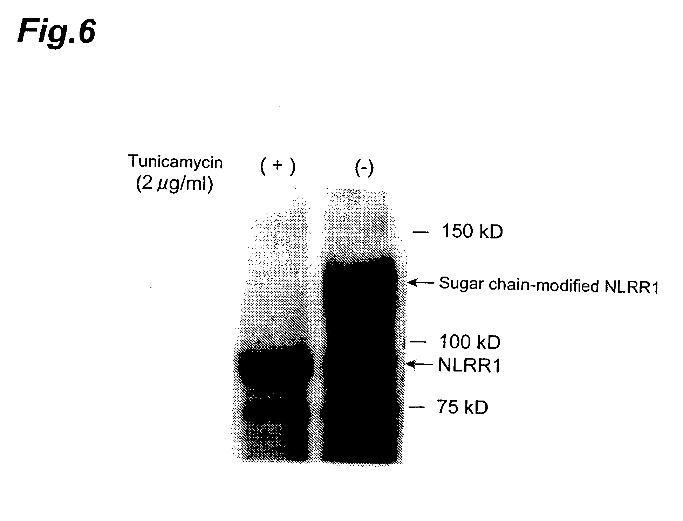

For the purpose of confirming the presence or absence of sugar chain modification, a plasmid encoding NLRR1 tagged with a myc tag was introduced into COS7 cells, the cells were cultured for 24 hours in the presence and absence of 2 µg/ml tunicamycin as a sugar chain synthesis inhibitor. After the cells were collected, the expression of NLRR1 was detected by an immunoblot method to the myc tag. Figure 6 is a photograph showing a result of an immunoblot. As a result, it has been clarified that many of NLRR1 proteins are subjected to sugar chain modification.

### (Example 5)

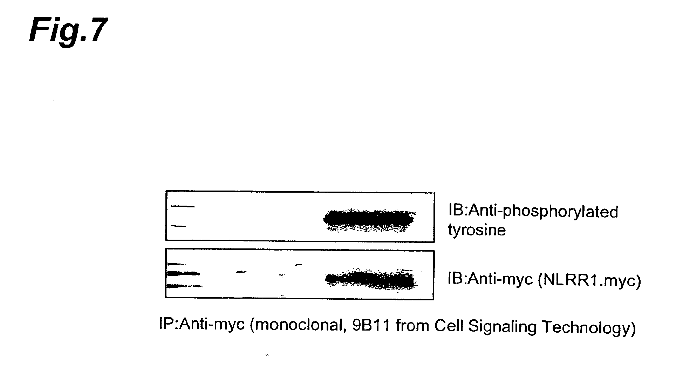

A plasmid encoding NLRR1 tagged with a myc tag was introduced into COS7 cells and cultured for 48 hours. The cells were collected and were subjected to immunoprecipitation using an anti-myc antibody. Next, samples were subjected to an SDS-polyacrylamide gel electrophoresis, and transferred to a membrane, followed by the detection by an immunoblot method using an anti-myc antibody and an anti-phosphorylated tyrosine antibody. Figure 7 is a photograph showing a result of an immunoblot. As a result, it is shown that a tyrosine residue of NLRR1 is phosphorylated. From the above, it is shown that when the cells are forced to express NLRR1, self-activation occurs, so that a tyrosine residue in an intracellular domain is phosphorylated.

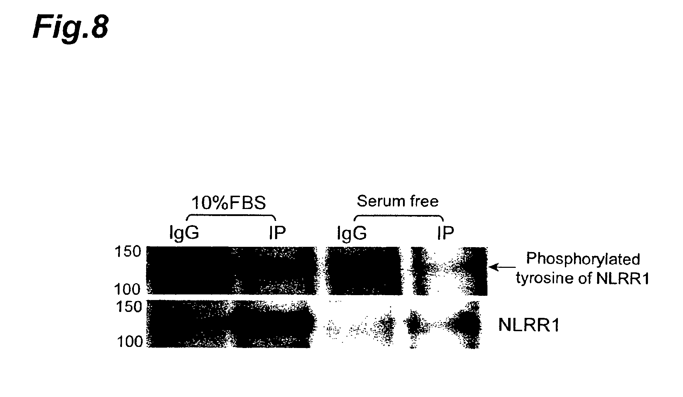

A plasmid encoding NLRR1 tagged with a myc tag was introduced into COS7 cells, and the cells were cultured for 48 hours in the condition containing 10% fetal bovine serum (FBS) and in the serum free condition. The cells were collected and were subjected to immunoprecipitation using an anti-myc antibody. Furthermore, as a control, immunoprecipitation using a standard mouse IgG was also carried out. Next, samples were subjected to an SDS-polyacrylamide gel electrophoresis, and transferred to a membrane, followed by detection by an immunoblot method using an anti-phosphorylated tyrosine antibody. Next, the membrane was subjected to reprobing and detected by an immunoblot method using an anti-myc antibody. Figure 8 is a photograph showing a result of the immunoblot. As a result, it is shown that a tyrosine residue of NLRR1 is phosphorylated both in the presence of serum and in the absence of serum. From the above, it is also shown that when cells are forced to express NLRR1, self-activation occurs, so that a tyrosine residue in an intracellular domain is phosphorylated.

### (Example 6)

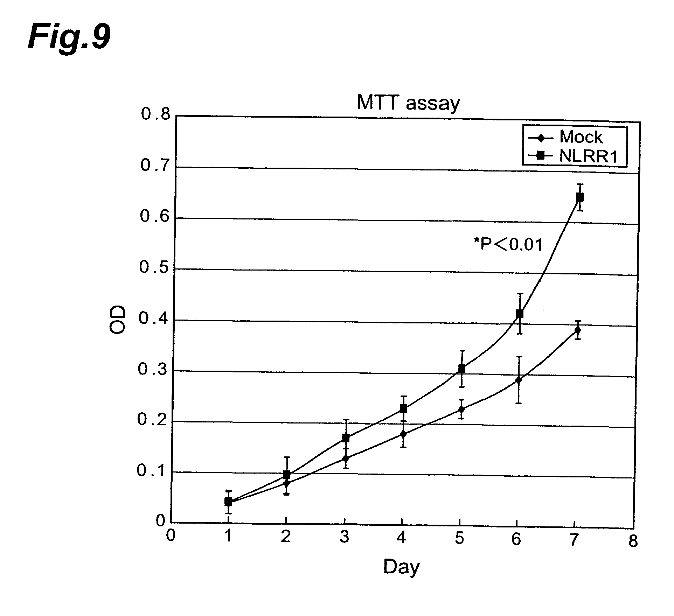

A plasmid encoding NLRR1 tagged with a myc tag was introduced into SK-N-BE cells derived from neuroblastoma, and the cells were forced to express. The proliferation of cells was measured by using a MTT assay kit. As a control, SK-N-BE cells into which an empty vector plasmid was introduced were used and the same measurement was carried out (which corresponds to "mock" in Figure 9). Figure 9 is a graph showing the proliferation of cells. As a result, it has been shown that with the forced expression of NLRR1, the proliferation of cells is promoted.

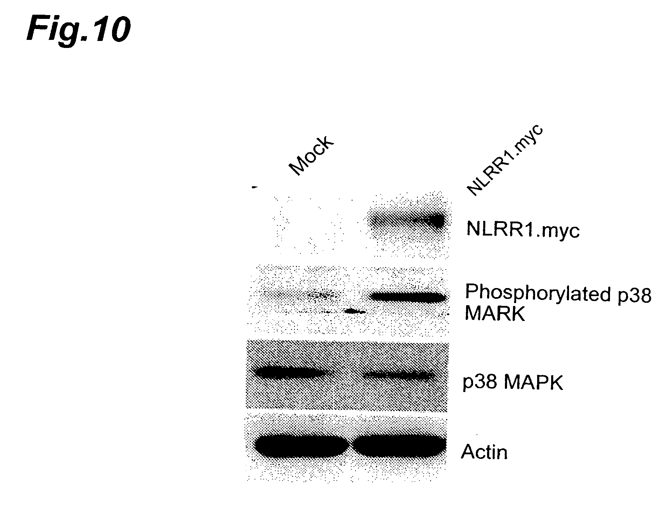

SK-N-BE cells into which NLRR1 tagged with a myc tag was introduced and SK-N-BE cells into which an empty vector plasmid was introduced (which corresponds to "mock" in Figure 10) were collected, followed by detection by the immunoblot method using an anti-myc antibody, an anti-phosphorylated p38MAPK antibody, and anti-p38MAPK antibody, and using an anti-actin antibody as a loading control. Figure 10 is a photograph showing a result of an immunoblot. As a result, it is shown that phosphorylation of p38MAPK that is a signal transduction molecule in the downstream is induced and activated.

### (Example 7)

To SK-N-BE cells derived from neuroblastoma, siRNA against NLRR1 was introduced so as to suppress the expression of NLRR1. As the siRNA, RNA fragment of the base sequences shown SEQ ID NOs: 3 and 4 were annealed and used. 5'-ucuugguugagcuguguaguu-3' (SEQ ID NO: 3) 5'-aacuacacagcucaaccaaga-3' (SEQ ID NO: 4)

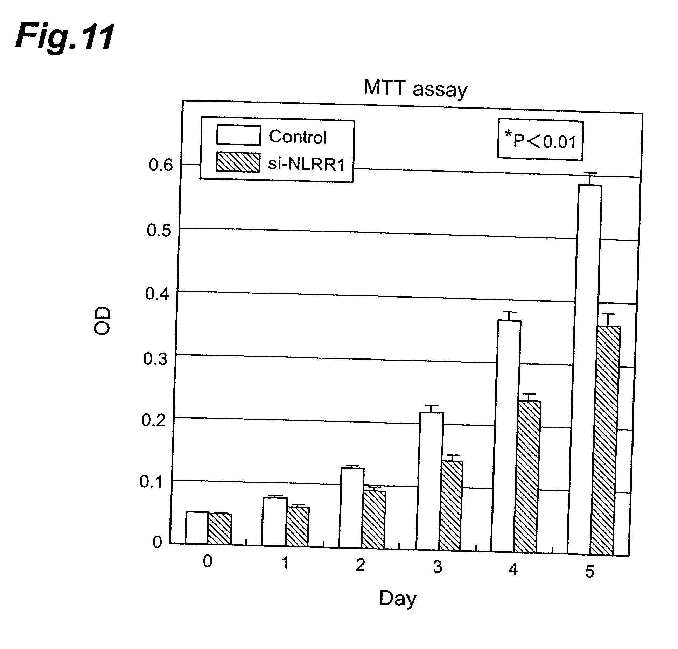

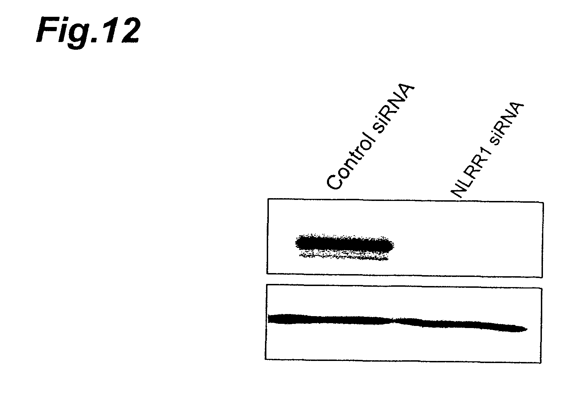

After siRNA was introduced, the cells were cultured, and the cell proliferation was quantified by the MTT assay over time. As a control, cells into which Silencer (registered trademark) Negative Control #1 siRNA (Ambion) was introduced were used. Figure 11 is a graph showing the proliferation of cells. As a result, it has been shown that by suppressing the expression of NLRR1, the proliferation of cells is suppressed. According to the results on day 4 and day 5, from the T-test, statistically significant differences were observed with the significance level of p < 0.01. Figure 12 is a photograph of an immunoblot in which the expression of NLRR1 in the cells into which siRNA against NLRR1 was introduced and the control cells were detected by using an anti-NLRR1 rabbit polyclonal antibody. The anti-NLRR1 rabbit polyclonal antibody was prepared by immunizing a rabbit with a synthetic peptide corresponding to 693 - 712 residues of an amino acid sequence of NLRR1 (SEQ ID NO: 2) as an antigen. The result when an anti-actin antibody was used as the loading control is also shown. It has been confirmed that the expression of NLRR1 was suppressed by the introduction of siRNA.

### (Example 8)

A plasmid encoding NLRR1 tagged with a myc tag was introduced into SK-N-AS cells. The SK-N-AS cells are a cell line which is derived from neuroblastoma and in which a MYCN oncogene is not amplified. After introduction of the gene, drug selection was carried out in a medium containing G418, and survival cells were cloned, and thereby a cell line continuously expressing NLRR1 was obtained. As a control cell line, cells subjected to the same operation by using an empty vector plasmid were used.

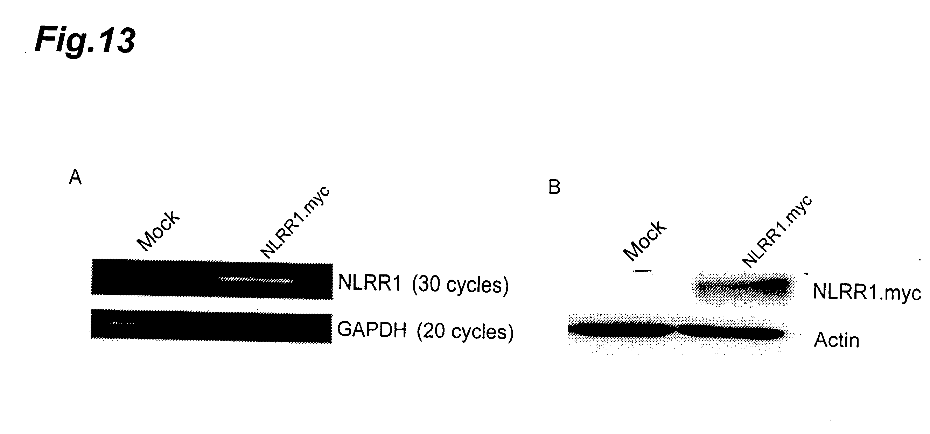

Figure 13A shows a photograph in which expression of mRNA of NLRR1 is confirmed by RT-PCR using control cells (corresponding to "mock") and a cell line continuously expressing NLRR1. As a control, mRNA of glyceraldehyde 3-phosphate dehydrogenase (GAPDH) was subjected to RT-PCR amplification. As a primer, primers having the following sequences were used: NLRR1 forward: 5'-aattcgtccctggtttaccc-3' (SEQ ID NO: 5), NLRR1 reverse: 5'-agcccgacctccttaatgtt-3' (SEQ ID NO: 6), GAPDH forward: 5'-acctgacctgccgtctagaa-3' (SEQ ID NO: 7), and GAPDH reverse: 5'-tccaccaccctgttgctgta-3' (SEQ ID NO: 8). The PCR reaction was carried out for 30 cycles in the detection of NLRR1, and 20 cycles in the detection of GAPDH. As a result, the expression of mRNA of the NLRR 1 gene was confirmed.

Figure 13B shows a result for detection of an immunoblot method of control cells (corresponding to "mock") and a cell line continuously expressing NLRR1 using an anti-myc antibody and an anti-actin antibody. As a result, an expression of NLRR1 in the protein level was confirmed.

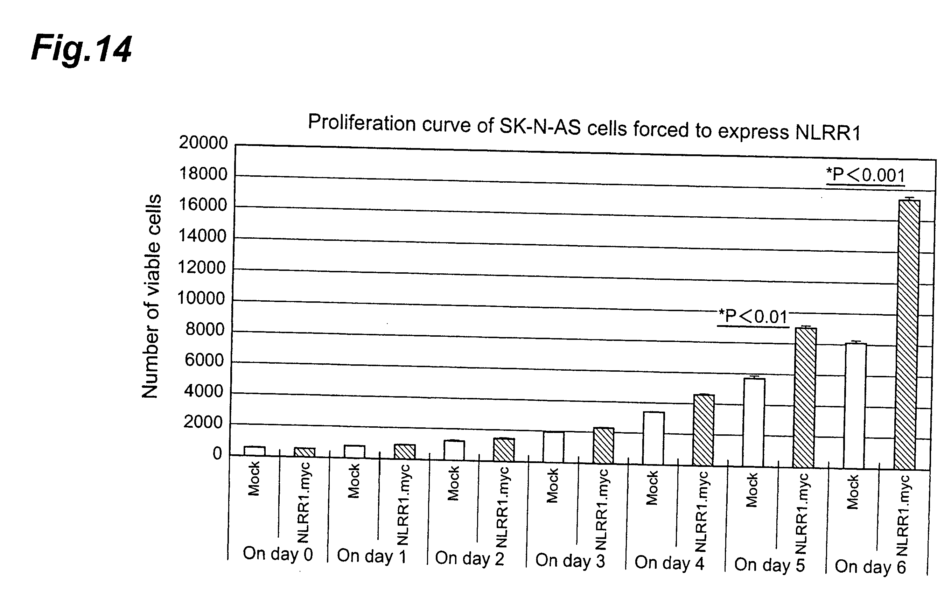

Figure 14 is a graph showing the proliferation of control cells (corresponding to "mock") and the cell line continuously expressing NLRR1. As a result, it has been shown that by the forced expression of NLRR1, the proliferation of cells is promoted. In the result, on day 5, from T-test, statistically significant difference was observed with the significance level of p < 0.01, and in the result on day 6, statistically significant difference was observed with the significance level of p < 0.001.

### (Example 9)

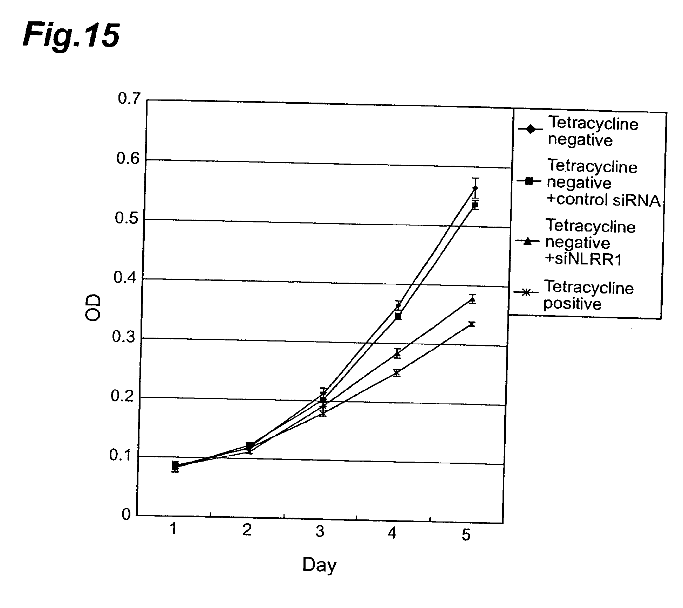

To SHEP21N cells that were forced to express MYCN in the absence of tetracycline, siRNA against NLRR1 was introduced, and the cell proliferation was quantified by the MTT assay over time. Figure 15 is a graph showing the proliferation of cells. In the absence of tetracycline (expression of MYCN was increased), the proliferation of cells was promoted as compared with the case in the presence of tetracycline (expression of MYCN was suppressed). When Silencer (registered trademark) Negative Control #1 siRNA was introduced in the absence of tetracycline (expression of MYCN was increased) as a control, the proliferation rate of cells was not changed. On the other hand, when siRNA against NLRR1 was introduced in the absence of tetracycline (expression of MYCN was increased), the proliferation of cells due to the increase in the MYCN expression did not occur. This suggests that NLRR1 functions in the downstream of MYCN.

### (Example 10)

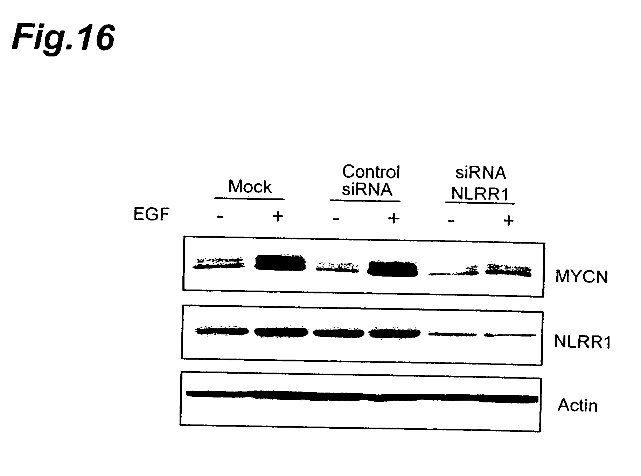

SK-N-BE cells, SK-N-BE cells into which siRNA against NLRR1 was introduced, and SK-N-BE cells into which Silencer (registered trademark) Negative Control #1 siRNA was introduced were cultured for 10 hours in the presence of 10 ng/ml EGF, and the cells were collected. Next, these cells were subjected to an immunoblot method using an anti-MYCN antibody, an anti-NLRR1 rabbit polyclonal antibody and an anti-actin antibody, and expression of MYCN and NLRR1 in the protein level was detected. Figure 16 is a photograph showing a result of immunoblot. The result of the cells into which control siRNA was introduced corresponds to "mock" in Figure 16. With the introduction of siRNA against NLRR1, the induction of the expression of MYCN by EGF treatment was suppressed. This result suggests that the induction of the expression of MYCN by EGF treatment is surely regulated by the expression amount of NLRR1.

### (Example 11)

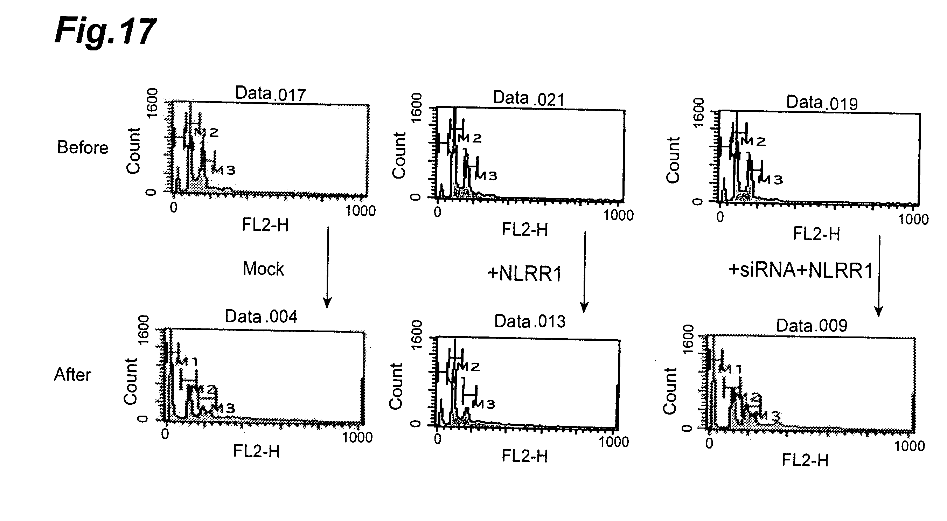

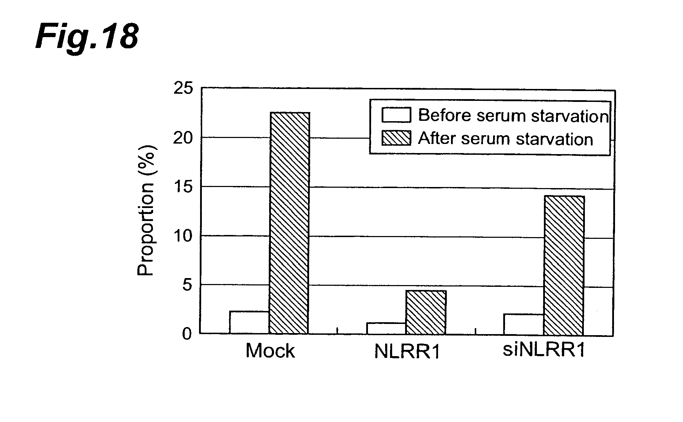

It is known that in the SK-N-AS neuroblastoma cell line, cell death is induced when serum is removed, and that the number of sub-G 1 cells is increased from the flow cytometry analysis. SK-N-AS cells into which an empty vector plasmid was introduced (corresponding to "mock" on the left in Figure 17), a cell line continuously expressing NLRR1 obtained by introducing a plasmid encoding NLRR1 into SK-N-AS cells (corresponding to "+NLRR1" in the middle of Figure 17), and cells obtained by introducing siRNA against NLRR1 into this cell line continuously expressing NLRR1 (corresponding to "+siRNA+NLRR1" on the right of Figure 17) were PI stained before and after the cells were cultured under the serum-free conditions for 36 hours, and analyzed by flow cytometry. The rate of the sub-G1 cells was evaluated as a rate of apoptosis. Figure 17 shows the results of the flow cytometry. Figure 18 is a graph showing the proportion of the number of sub-G1 cells to the total number of cells before and after culturing under the serum free conditions of respective cells based on the results of Figure 17, in which the unit of the ordinate axis is %. In the SK-N-AS cells, not less than 20% of cells showed apoptosis, but in the cell line continuously expressing NLRR1, the rate was reduced. Furthermore, in the cells obtained by intruding siRNA against NLRR1 into the cell line continuously expressing NLRR1, the increase in the apoptosis was observed. This suggests that NLRR1 is deeply involved in a molecular mechanism for suppressing the apoptosis by the removal of serum.

### (Example 12)

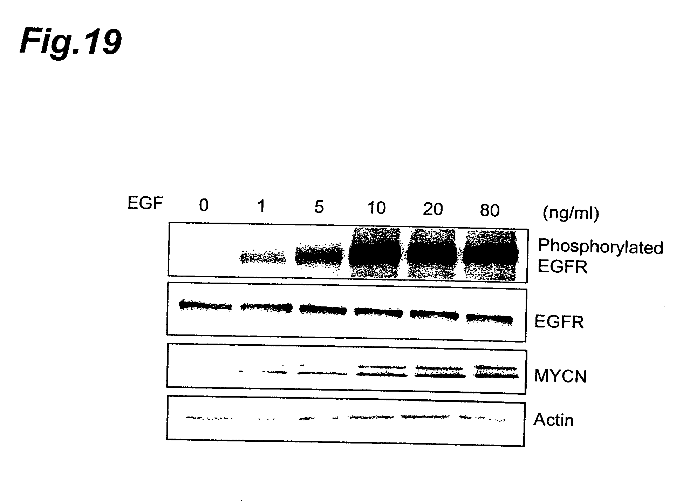

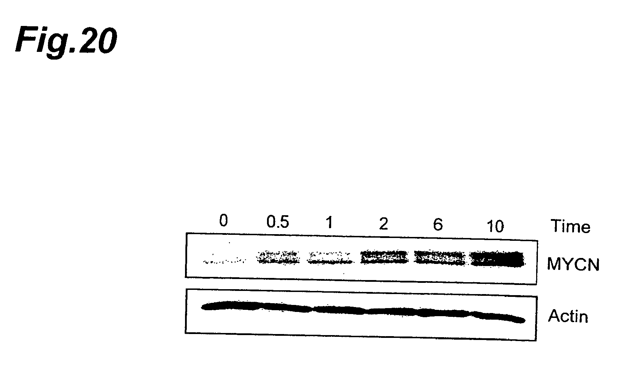

A neuroblastoma cell line SK-N-BE was cultured for 10 hours in media containing EGF at a variety of concentrations, by using an anti-phosphorylated EGFR antibody, an anti-EGFR antibody, an anti-MYCN antibody and an anti-actin antibody, the phosphorylation of an EGF receptor and the expression of MYCN were analyzed by an immunoblot method. Figure 19 is a photograph showing a result of immunoblot. It has been shown that the expression of MYCN is increased depending upon the concentration of EGF. Figure 20 is a photograph in which SK-N-BE cells were cultured in a medium containing 10 ng/ml EGF for different periods of time and the expression of MYCN was analyzed by an immunoblot method using an anti-MYCN antibody and an anti-actin antibody. Increased expression of MYCN by EGF over time was shown. From the above-mentioned results, it is thought that the following positive feed-back mechanism is present. That is to say, by EGF treatment, an EGFR signal is activated and the expression of MYCN is promoted. Next, by MYCN, transcription of NLRR1 is promoted. Next, the expression of NLRR1 is increased and activated. Next, by NLRR1, an EGFR signal is activated. Next, the expression of MYCN is further promoted.

### (Example 13)

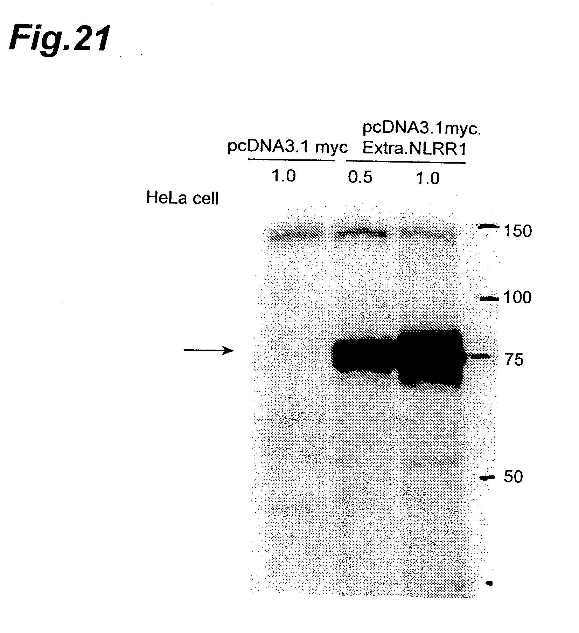

An expression plasmid (pcDNA3.1myc.Extra.NLRR1) encoding only an extracellular domain of NLRR1 and an expression plasmid (pcDNA3.1Cyt.NLRR1.myc) encoding only an intracellular domain were prepared. Both plasmids were tagged with a myc tag. To HeLa cells, 0.5 µg and 1.0 µg of pcDNA3.1myc.Extra.NLRR1 were introduced, respectively, followed by detection by an immunoblot method using an anti-Myc antibody. As a control, HeLa cells into which 1.0 µg of empty vector plasmid was introduced were used. Figure 21 is a photograph of an immunoblot in which the expression in an extracellular domain of NLRR1 is detected. As a result, the expression in an extracellular domain of NLRR1 was confirmed.

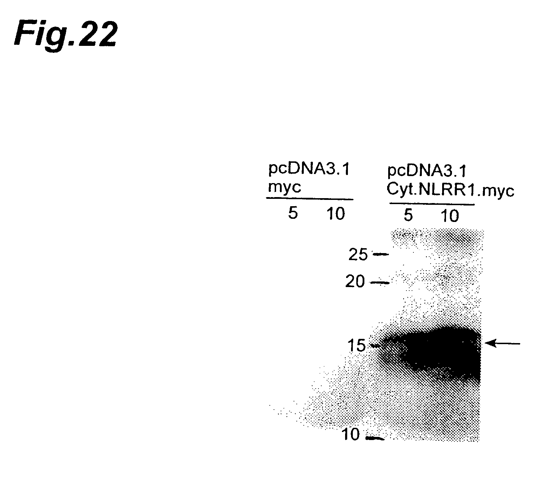

5 µg and 10 µg of pcDNA3.1Cyt.NLRR1.myc were allowed to express, respectively. For expression, In vitro Transcription/Translation System was used. As a control, 5 µg and 10 µg of empty vector plasmids were allowed to express. Figure 22 is a photograph of immunoblot in which the expression in an intracellular domain of NLRR1 is detected. As a result, the expression in an intracellular domain of NLRR1 was observed.

### (Example 14)

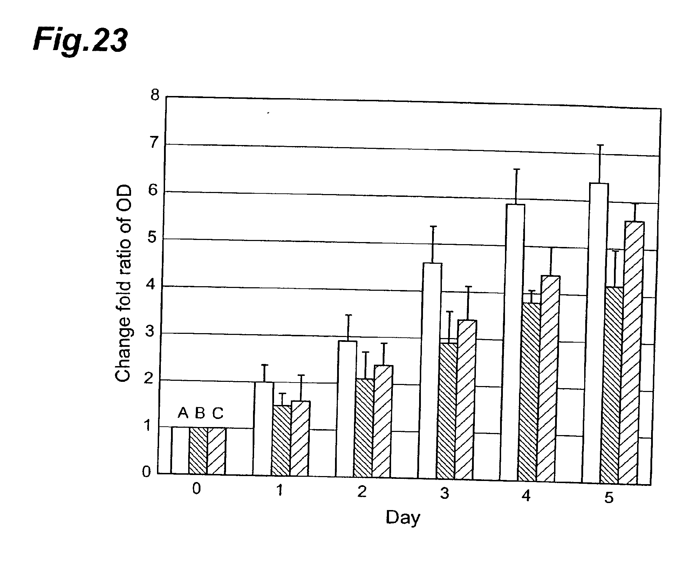

SK-N-BE cells that were forced to express NLRR1 were cultured in the presence and absence of ERK1/2 (ERK1 and ERK2) inhibitor, U0126. Furthermore, as a control, SK-N-BE cells into which a plasmid encoding NLRR1 was not introduced were cultured in the absence of U0126. The proliferation of these cells was quantified by an MTT assay over time. Figure 23 is a graph showing a proliferation of cells. In the drawing, A denotes the proliferation of SK-N-BE cells forced to express NLRR1; B denotes the proliferation of SK-N-BE cells forced to express NLRR1 in the presence of U0126; and C denotes the proliferation of control cells. As a result, by the ERK1/2 inhibitor, the cell proliferation was suppressed.

### (Example 15)

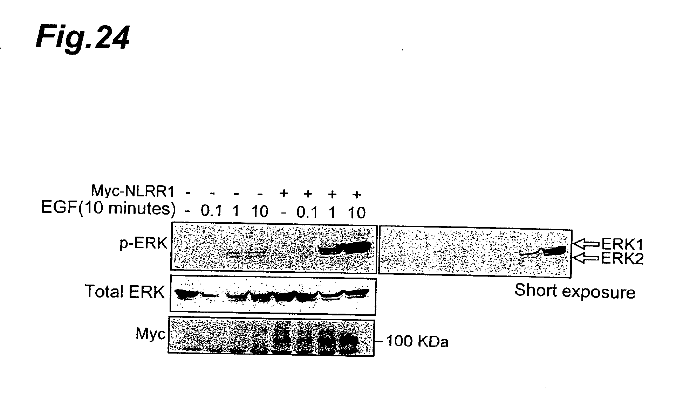

A breast cancer cell line MCF-7 was forced to express NLRR1, which was cultured in media containing 0, 0.1, 1 and 10 ng/ml EGF for 10 minutes. Next, cells were collected and subjected to an immunoblot method using an anti-phosphorylated ERK antibody, an anti-ERK antibody, and an anti-myc antibody. Figure 24 is a photograph showing a result of an immunoblot. As a result, it has been clarified that the expression of NLRR1 enhances the phosphorylation of ERK by EGF.

### (Example 16)

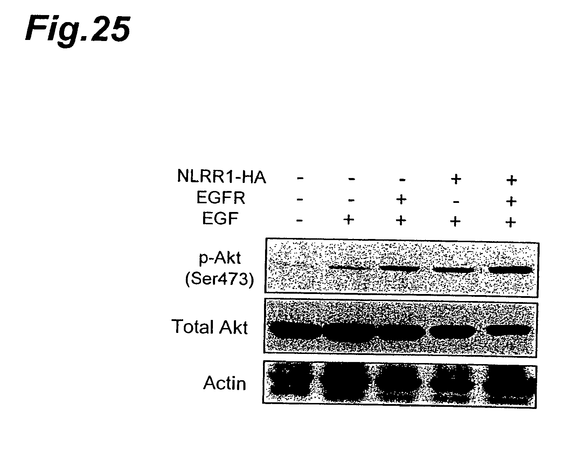

A combination of a plasmid encoding only NLRR1, a plasmid encoding only EGFR, as well as a plasmid encoding NLRR1 and EGFR were introduced into MCF-7 cells and were expressed. As a control, MCF-7 cells into which genes were not introduced were used. These cells were cultured in a medium containing 1 ng/ml EGF for 10 minutes. Next, the cells were collected and subjected to an immunoblot method using an anti-phosphorylated-Akt antibody, an anti-Akt antibody and an anti-actin antibody. Figure 25 is a photograph showing a result of an immunoblot. As a result, it has been shown that the expression of NLRR1 and EGFR enhances the phosphorylation of Akt by EGF. Furthermore, it has been clarified that co-expression of NLRR1 and EGFR further enhances the phosphorylation of Akt.

### (Example 17)

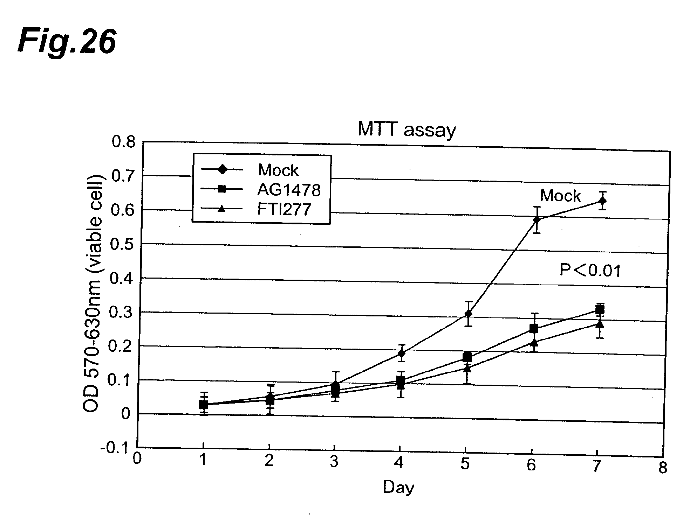

SK-N-BE cells were cultured in the presence of 10 ng/ml EGF (control, which corresponds to "mock" in Figure 26), in the presence of 10 ng/ml EGF and 5 µM of EGFR inhibitor AG1478, as well as in the presence of 10 ng/ml EGF and 5 µM of Ras inhibitor FTI277, and the cell proliferation was quantified by the MTT assay over time. Figure 26 is a graph showing the proliferation of cells. As a result, as compared with the control, the proliferation of cells is suppressed in the presence of AG1478 and FTI277, and from the T-test, statistically significant differences were observed with the significance level of p < 0.01.

### (Example 18)

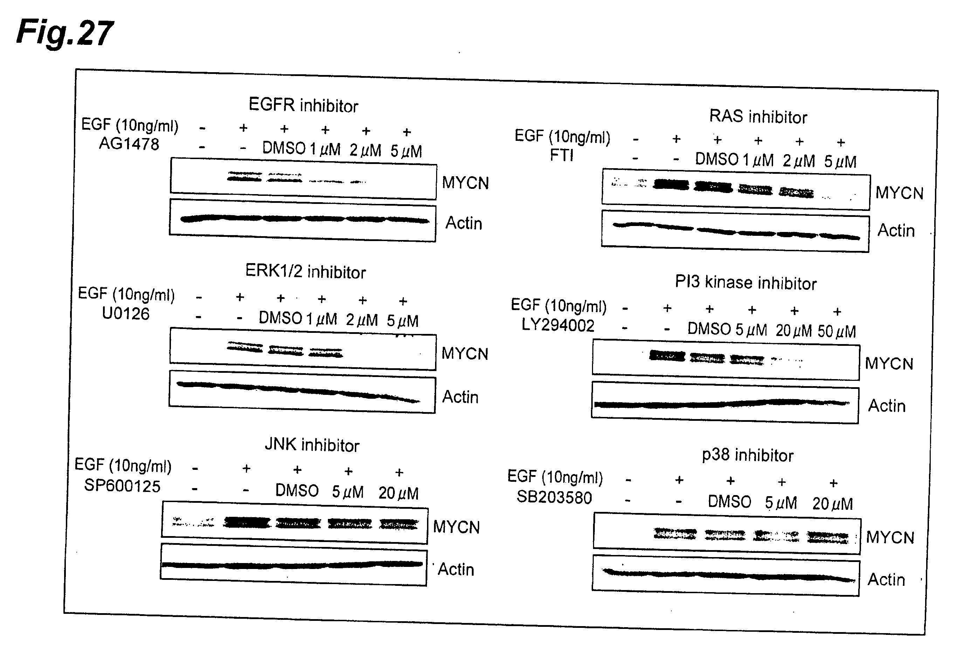

SK-N-BE cells were cultured for six hours in the presence of 10 ng/ml EGF and in the presence of an EGFR inhibitor AG1478, a Ras inhibitor FTI277, an ERK1/2 inhibitor U0126, a PI3Kinase inhibitor LY294002, a JNK inhibitor SP600125 or a p38MAPK inhibitor SB203580 in each concentration shown in Figure 27. Next, respective cells were collected, and the expression of MYCN was analyzed by an immunoblot method using an anti-MYCN antibody and an anti-actin antibody. Figure 27 is a photograph showing a result of an immunoblot. As a result, it has been shown that when EGFR, Ras, ERK1/2 and PI3Kinase were inhibited, expression increase of MYCN by the induction of EGF was inhibited. Furthermore, even when JNK and p38MAPK were inhibited, expression increase of MYCN by the induction of EGF was not inhibited. From the result, it has been clarified that expression increase of MYCN by the induction of EGF depends on the pathways of EGFR, Ras, ERK1/2 and PI3Kinase, but does not depend on JNK and p38MAPK.

### (Example 19)

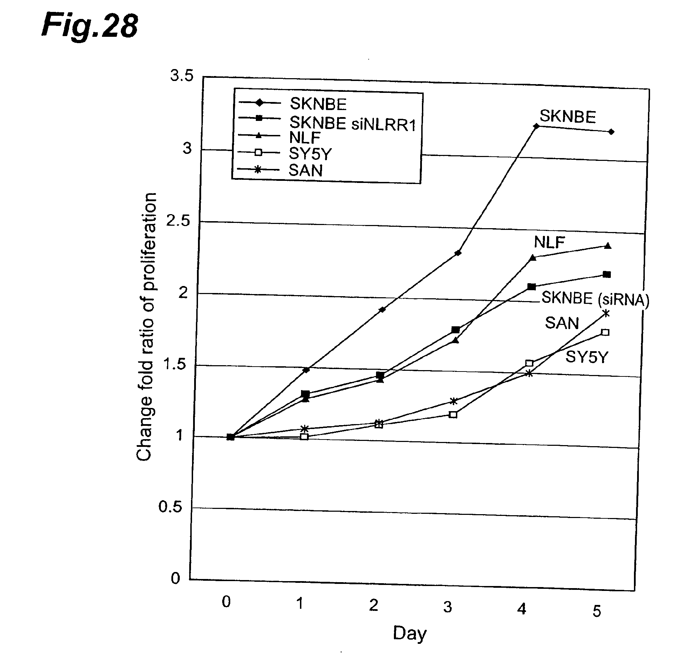

SK-N-BE cells, NLF cells, SH-SY5Y cells, SMS-SAN cells, which are cell lines derived from neuroblastoma, and SK-N-BE cells into which siRNA against NLRR1 was introduced were cultured in the presence and absence of 10 ng/ml EGF, and the cell proliferation was quantified by the MTT assay over time. Figure 28 is a graph showing the cell proliferation, showing the change fold ratio of the proliferation of cells in the presence of EGF to the proliferation of cells in the absence of EGF. As a result, it has been shown that the proliferation of cells derived from neuroblastoma is promoted by EGF. Furthermore, by suppressing the expression of NLRR1, EGF-induced cell proliferation was remarkably suppressed. This result shows that NLRR1 plays an important role in EGF-induced cell proliferation in neuroblastoma.

### (Example 20)

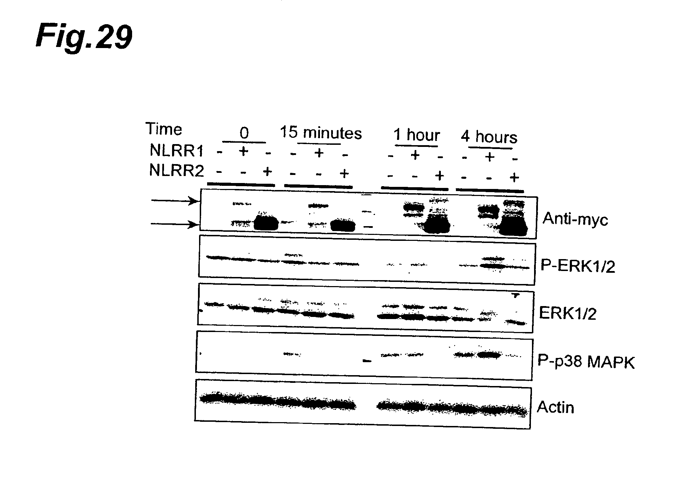

COS7 cells were forced to express NLRR1 (tagged with a myc tag) or NLRR2 (tagged with a myc tag) that is one of the NLRR family genes. The cells were subjected to serum starvation for 24 hours; 0.02 ng/ml EGF was then added to the medium; and the cells were collected after 0 minute, 15 minutes, 1 hour, and 4 hours. These cells were subjected to an immunoblot using an anti-myc antibody, an anti-phosphorylated ERK antibody, an anti-ERK antibody, an anti-phosphorylated p38MAPK antibody and an anti-actin antibody. Figure 29 is a photograph showing a result of an immunoblot. As a result, in COS7 cells into which NLRR1 had been introduced, phosphorylation of p38MAPK was observed.

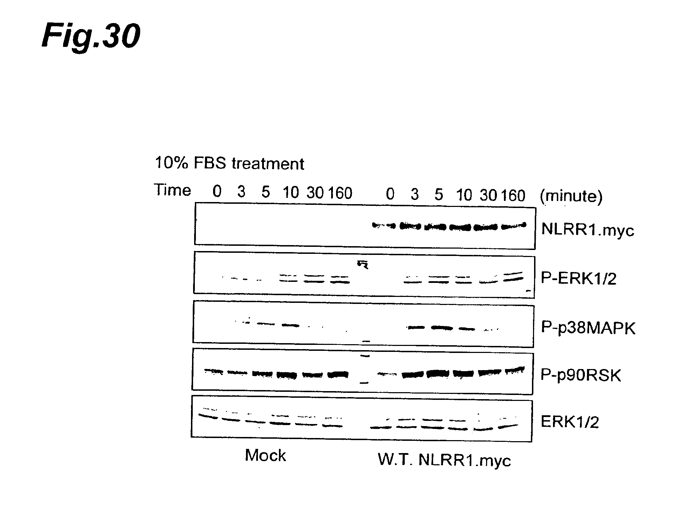

SK-N-AS cells were forced to express NLRR1 tagged with a myc tag. The cells were subjected to serum starvation for 12 hours; 10% fetal bovine serum (FBS) was then added into a medium; and the cells were collected after 0, 3, 5, 10, 30 and 160 minutes. As a control, SK-N-AS cells into which an empty vector plasmid was introduced was used (which corresponds to "mock" in Figure 30). These cells were subjected to an immunoblot using an anti-myc antibody, an anti-phosphorylated-ERK antibody, an anti-ERK antibody, an anti-phosphorylated p38MAPK antibody, and an anti-phosphorylated p90RSK antibody. Figure 30 is a photograph showing a result of an immunoblot. As a result, it has shown that by the serum treatment, p38MAPK was phosphorylated, and this phosphorylation was enhanced by the forced expression of NLRR1. Furthermore, similarly, it has been shown that by the serum treatment, ERK1/2 was phosphorylated and this phosphorylation was enhanced by the forced expression of NLRR1.

From the above-mentioned results, it has been clarified that NLRR1 promotes the phosphorylation of p38MAPK not only in cells derived from neuroblastoma but also in COS7 cells.

### (Example 21)

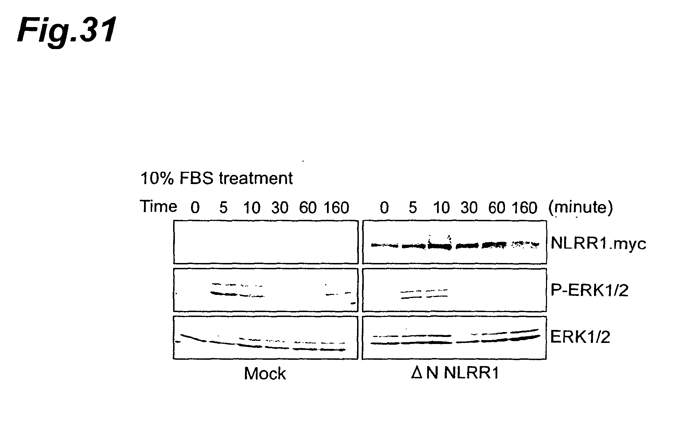

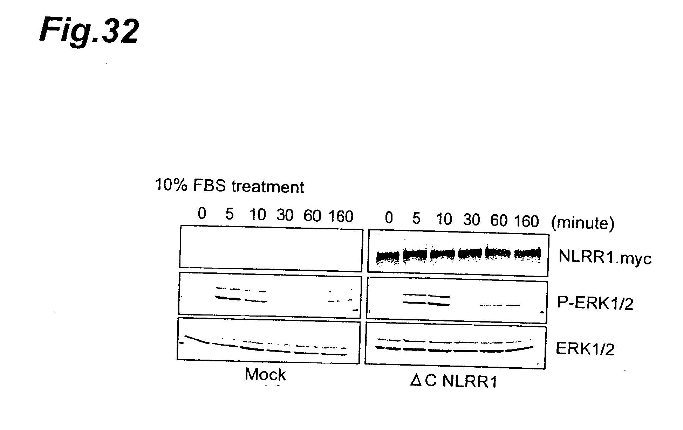

An expression plasmid encoding only an intracellular domain of NLRR1 (tagged with a myc tag) or only an extracellular domain (tagged with a myc tag) were introduced into SK-N-BE cells. As a control, cells into which an empty vector plasmid was introduced were used (which corresponds to "mock" in Figures 31 and 32). These cells were subjected to serum starvation for 12 hours; 10% fetal bovine serum (FBS) was then added to the medium; and the cells were collected after 0, 5, 10, 30, 60 and 160 minutes. These cells were subjected to an immunoblot using an anti-myc antibody, an anti-phosphorylated-ERK antibody and an anti-ERK antibody. Figure 31 is a photograph showing a result of an immunoblot of cells into which only an intracellular domain of NLRR1 was introduced. Figure 32 is a photograph showing a result of an immunoblot of cells into which only an extracellular domain of NLRR1 was introduced. As a result, in any deletion mutants, it has been shown that the activation of ERK1/2 cannot be enhanced as compared with the control.

### (Example 22)

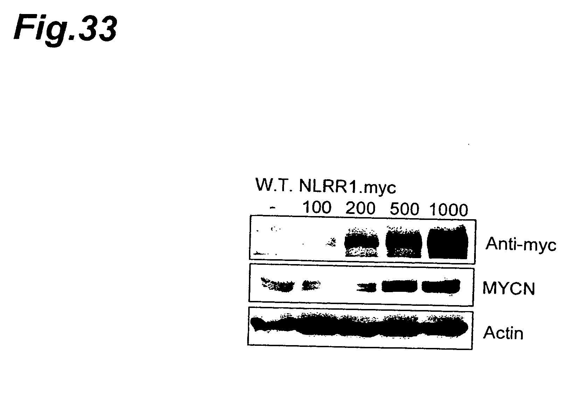

An expression plasmid of NLRR1 tagged with a myc was introduced into SK-N-BE cells at the concentrations of 0, 100, 200, 500, and 1000 ng/ml. After 48 hours of culturing, the cells were collected and were subjected to an immunoblot method using an anti-myc antibody, an anti-MYCN antibody and an anti-actin antibody. Figure 33 is a photograph showing a result of an immunoblot. As a result, the induction of the expression of MYCN protein according to the expression amount of NLRR1 was observed.

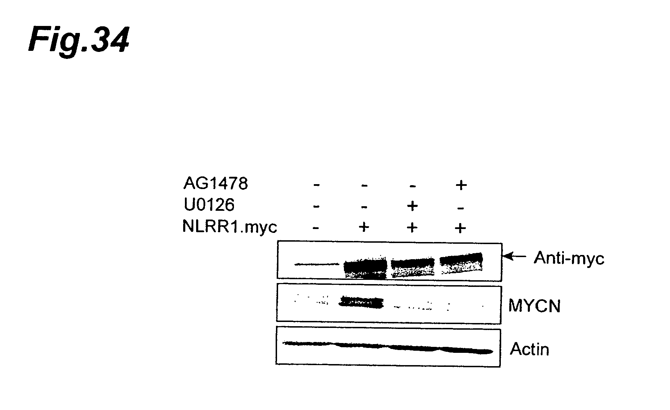

An expression plasmid of NLRR1 tagged with a myc tag was introduced into SK-N-BE cells at the concentration of 1000 ng/ml. The cells were cultured for 12 hours in the presence of 1 µM of EGFR inhibitor AG1478 or 2 µM of ERK1/2 inhibitor U0126, and then the cells were collected and subjected to an immunoblot method using an anti-myc antibody, an anti-MYCN antibody and an anti-actin antibody. Figure 34 is a photograph showing a result of an immunoblot. As a result, it has been shown that expression induction of MYCN protein by the expression of NLRR1 is suppressed in the presence of the EGFR inhibitor AG1478 or the ERK1/2 inhibitor U0126.

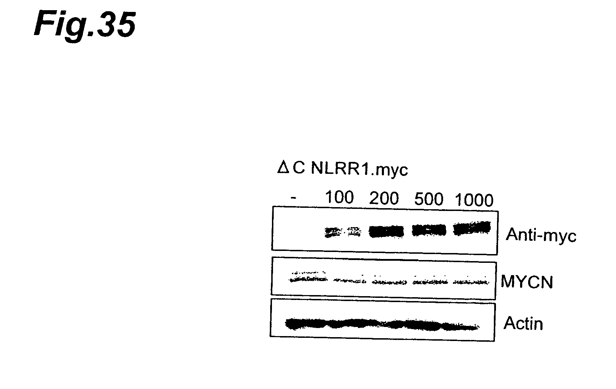

An expression plasmid encoding only an extracellular domain of NLRR1 tagged with a myc tag was introduced into SK-N-BE cells at the concentrations of 0, 100, 200, 500, and 1000 ng/ml. The cells were cultured for 48 hours, and then the cells were collected and subjected to an immunoblot method using an anti-myc antibody, an anti-MYCN antibody and an anti-actin antibody. Figure 35 is a photograph showing a result of the immunoblot. As a result, it has shown that with only an extracellular domain of NLRR1, the expression of MYCN protein was not induced.

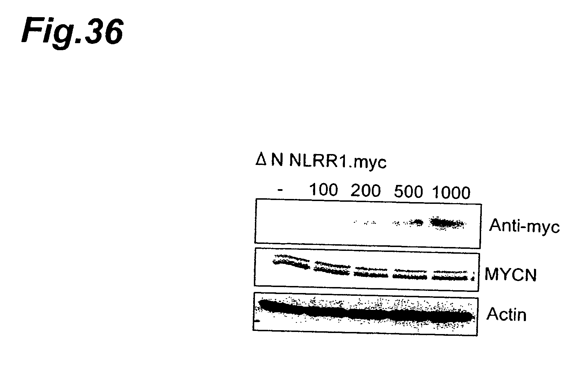

An expression plasmid encoding only an intracellular domain of NLRR1 tagged with a myc tag was introduced into SK-N-BE cells at the concentrations of 0, 100, 200, 500, and 1000 ng/ml. The cells were cultured for 48 hours, and then the cells were collected and subjected to an immunoblot method using an anti-myc antibody, an anti-MYCN antibody and an anti-actin antibody. Figure 36 is a photograph showing a result of the immunoblot. As a result, it has shown that with only an intracellular domain of NLRR1, the expression of MYCN protein was not induced.

From the above-mentioned results, it has been clarified that when either an extracellular domain or an intracellular domain of NLRR1 is absent, the expression of MYCN protein in the downstream cannot be induced.

### (Example 23)

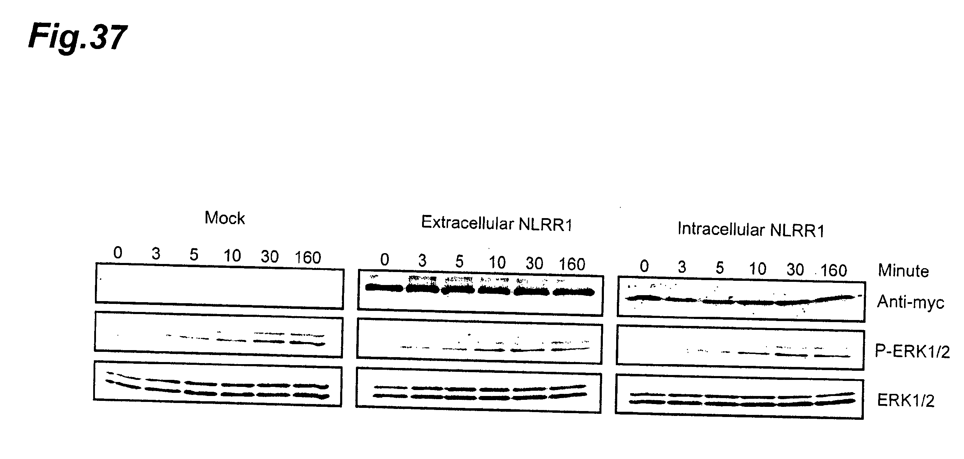

An expression plasmid encoding only an extracellular domain of NLRR1 (tagged with a myc tag) or only an intracellular domain (tagged with a myc tag) was introduced into SK-N-BE cells. As a control, cells into which an empty vector plasmid was introduced were used (which corresponds to "mock" in Figure 37). These cells were subjected to serum starvation for 12 hours; 10% FBS was added into a medium; and the cells were collected after 0, 3, 5, 10, 30 and 160 minutes. These cells were subjected to an immunoblot using an anti-myc antibody, an anti-phosphorylated-ERK antibody and an anti-ERK antibody. Figure 37 is a photograph showing a result of an immunoblot. As a result, it was shown that neither deletion mutant with only an extracellular domain nor only an intracellular domain of NLRR1 can enhance the activation of ERK1/2 as compared with the control.

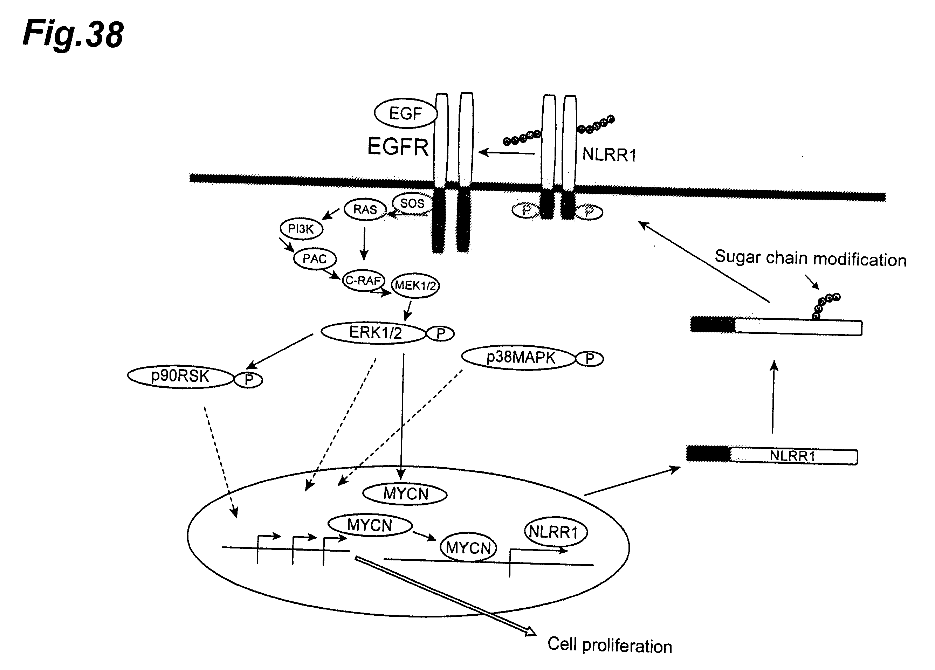

Figure 38 is a view showing a regulation of NLRR1 by MYCN, which the present inventors has clarified. With EGF treatment, the EGFR signal is activated, and the expression of MYCN is promoted. Then, with MYCN, the expression of NLRR1 is promoted. NLRR1 undergoes sugar chain modification. When the expression of NLRR1 is increased, activation occurs, and tyrosine residues in an intracellular domain are phosphorylated. NLRR1 is bound to EGFR, and enhances the EGFR signal by EGF. Thus, the expression of MYCN is further promoted.

### (Example 24)

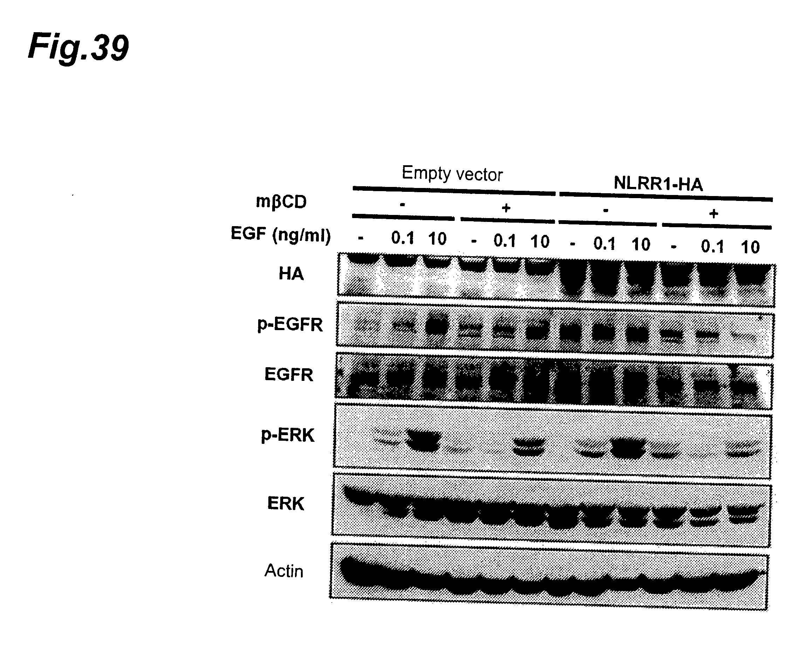

The effect of NLRR1 on EGF signal transduction in cells treated with cyclodextrin (methyl-β-cyclodextrin, mβCD) that inhibits lipid raft was examined. Figure 39 is a photograph showing a result of an immunoblot. MCF-7 cells that were forced to express NLRR1 were cultured in a medium containing cyclodextrin (10 mM) for 30 minutes at 37°C. The cells were washed with the medium, and cultured for three minutes in a medium containing EGF with different concentrations. Then, the phosphorylation of EGFR and ERK was analyzed by Western blotting. With the expression of NLRR1, the enhancement of phosphorylation of EGFR and ERK by EGF was detected. On the contrary, when the cells were treated with mβCD so as to remove cholesterol and to break lipid raft in advance, the enhancement of phosphorylation of EGFR and ERK by NLRR1 was not observed. From the result, it has been clarified that the enhancement of an EGF signal by NLRR1 needs the structure of lipid raft. Thus, it is thought that treatment of cancer using the combination of an antibody inhibiting NLRR1 and an inhibitor of lipid raft is effective.

### Industrial Applicability

According to the present invention, a screening method for a therapeutic agent for cancer based on a new mechanism is provided.

## Claims

1. A kit for measuring a binding of neuronal Leucine-rich repeat protein 1 (NLRR1) and epidermal growth factor receptor (EGFR) comprising an antibody recognizing NLRR1 and an antibody recognizing EGFR,
wherein the NLRR1 consists of the amino acid sequence as set forth in SEQ ID NO: 2.

2. A screening method for a therapeutic agent for cancer, the method comprising:
a step of measuring a binding of NLRR1 and EGFR under each condition of being in the presence of a test substance and in the absence of a test substance; and
a step of determining that the test substance is a therapeutic agent for cancer when the binding of NLRR1 and EGFR in the presence of the test substance is weaker than the binding of NLRR1 and EGFR in the absence of the test substance,
wherein the NLRR1 consists of the amino acid sequence as set forth in SEQ ID NO: 2.

3. A screening method for a therapeutic agent for cancer, the method comprising:
a step of culturing cells expressing NLRR1 and EGFR under each condition of being in the presence of a test substance and in the absence of a test substance;
a step of measuring a binding of NLRR1 and EGFR in each cultured cells, and
a step of determining that the test substance is a therapeutic agent for cancer when the binding of NLRR1 and EGFR in the cells cultured in the presence of the test substance is weaker than the binding of NLRR1 and EGFR in the cells cultured in the absence of the test substance,
wherein the NLRR1 consists of the amino acid sequence as set forth in SEQ ID NO: 2.

## Patentansprüche

1. Kit um eine Bindung von Neuronal Leucine-Rich Repeat Protein 1 (NLRR1) und Epidermal Growth Factor Rezeptor (EGFR) zu messen, welches einen Antikörper, der NLRR1 erkennt und einen Antikörper, der EGFR erkennt, umfasst,
wobei das NLRR1 aus der Aminosäuresequenz besteht, die in SEQ ID NO:2 dargelegt ist.

2. Screening-Verfahren für ein Therapeutikum für Krebs, das Verfahren umfasst:
einen Schritt der Messung einer Bindung von NLRR1 und EGFR unter jeweils der Bedingung in Anwesenheit einer Testsubstanz und in Abwesenheit einer Testsubstanz zu sein; und
einen Schritt der Feststellung, dass die Testsubstanz ein Therapeutikum für Krebs ist, wenn die Bindung von NLRR1 und EGFR in Anwesenheit der Testsubstanz schwächer ist als die Bindung von NLRR1 und EGFR in Abwesenheit der Testsubstanz,
wobei das NLRR1 aus der Aminosäuresequenz besteht, die in SEQ ID NO:2 dargelegt ist.

3. Screening-Verfahren für ein Therapeutikum für Krebs, wobei das Verfahren umfasst:
einen Schritt der Kultivierung von Zellen, die NLRR1 und EGFR exprimieren, unter jeweils der Bedingung in Anwesenheit einer Testsubstanz und in Abwesenheit einer Testsubstanz zu sein;
einen Schritt eine Bindung von NLRR1 und EGFR in den jeweils kultivierten Zellen zu messen, und
einen Schritt der Feststellung, dass die Testsubstanz ein Therapeutikum für Krebs ist, wenn die Bindung von NLRR1 und EGFR in den, in Anwesenheit der Testsubstanz kultivierten Zellen schwächer ist als die Bindung von NLRR1 und EGFR in den, in Abwesenheit der Testsubstanz kultivierten Zellen,
wobei das NLRR1 aus der Aminosäuresequenz besteht, die in SEQ ID NO:2 dargelegt ist.

## Revendications

1. Kit pour mesurer la liaison de la protéine 1 de répétition riche en leucine neuronale (NLRR1) et du récepteur de facteur de croissance épidermique (EGFR) comprenant un anticorps reconnaissant NLRR1 et un anticorps reconnaissant EGFR, dans lequel NLRR1 est constituée de la séquence d'acides aminés telle que présentée dans la SEQ ID NO : 2.

2. Procédé de dépistage d'un agent thérapeutique contre le cancer, lequel procédé comprend :
une étape consistant à mesurer la liaison de NLRR1 et EGFR dans chacune des conditions suivantes : en présence d'une substance de test et en l'absence d'une substance de test ; et
une étape consistant à déterminer que la substance de test est un agent thérapeutique contre le cancer quand la liaison de NLRR1 et EGFR en présence de la substance de test est plus faible que la liaison de NLRR1 et EGFR en l'absence de la substance de test,
dans lequel NLRR1 est constituée de la séquence d'acides aminés telle que présentée dans la SEQ ID NO : 2.

3. Procédé de dépistage d'un agent thérapeutique contre le cancer, lequel procédé comprend :
une étape consistant à cultiver des cellules exprimant NLRR1 et EGFR dans chacune des conditions suivantes : en présence d'une substance de test et en l'absence d'une substance de test ;
une étape consistant à mesurer la liaison de NLRR1 et EGFR dans chacune des cellules cultivées, et
une étape consistant à déterminer que la substance de test est un agent thérapeutique contre le cancer quand la liaison de NLRR1 et EGFR dans les cellules cultivées en présence de la substance de test est plus faible que la liaison de NLRR1 et EGFR dans les cellules cultivées en l'absence de la substance de test,
dans lequel NLRR1 est constituée de la séquence d'acides aminés telle que présentée dans la SEQ ID NO : 2.


### Sequence Listing

<110> HISAMITSU PHARMACEUTICAL CO., INC. Chiba-Prefecture
<120> Method for screening of therapeutic agent for cancer
<130> EP72433FZ163pau
<140> EP 09 718 200.0
   <141> 2009-02-27
<150> PCT/JP2009/053699
   <151> 2009-02-27
<150> JP2008-053959
   <151> 2008-03-04
<160> 8
<170> PatentIn version 3.5
<210> 1
   <211> 3060
   <212> DNA
   <213> Homo sapiens
<220>
   <221> misc_feature
   <223> NLRR1 cDNA sequence
<400> 1
   atggctagga tgagctttgt tatagcagct tgccaattgg tgctgggcct actaatgact      60
   tcattaaccg agtcttccat acagaatagt gagtgtccac aactttgcgt atgtgaaatt     120
   cgtccctggt ttaccccaca gtcaacttac agagaagcca ccactgttga ttgcaatgac     180
   ctccgcttaa caaggattcc cagtaacctc tctagtgaca cacaagtgct tctcttacag     240
   agcaataaca tcgcgaagac tgtggatgag ctgcagcagc ttttcaactt gactgaacta     300
   gatttctccc aaaacaactt tactaacatt aaggaggtcg ggctggcaaa cctaacccag     360
   ctcacaacgc tgcatttgga ggaaaatcag attaccgaga tgactgatta ctgtctacaa     420
   gacctcagca accttcaaga actctacatc aaccacaacc aaattagcac tatttctgct     480
   catgcttttg caggcttaaa aaatctatta aggctccacc tgaactccaa caaattgaaa     540
   gttattgata gtcgctggtt tgattctaca cccaacctgg aaattctcat gatcggagaa     600
   aaccctgtga ttggaattct ggatatgaac ttcaaacccc tcgcaaattt gagaagctta     660
   gttttggcag gaatgtatct cactgatatt cctggaaatg ctttggtggg tctggatagc     720
   cttgagagcc tgtcttttta tgataacaaa ctggttaaag tccctcaact tgccctgcaa     780
   aaagttccaa atttgaaatt cttagacctc aacaaaaacc ccattcacaa aatccaagaa     840
   ggggacttca aaaatatgct tcggttaaaa gaactgggaa tcaacaatat gggcgagctc     900
   gtttctgtcg accgctatgc cctggataac ttgcctgaac tcacaaagct ggaagccacc     960
   aataacccta aactctctta catccaccgc ttggctttcc gaagtgtccc tgctctggaa    1020
   agcttgatgc tgaacaacaa tgccttgaat gccatttacc aaaagacagt cgaatccctc    1080
   cccaatctgc gtgagatcag tatccatagc aatcccctca ggtgtgactg tgtgatccac 1140
   tggattaact ccaacaaaac caacatccgc ttcatggagc ccctgtccat gttctgtgcc 1200
   atgccgcccg aatataaagg gcaccaggtg aaggaagttt taatccagga ttcgagtgaa 1260
   cagtgcctcc caatgatatc tcacgacagc ttcccaaatc gtttaaacgt ggatatcggc 1320
   acgacggttt tcctagactg tcgagccatg gctgagccag aacctgaaat ttactgggtc 1380
   actcccattg gaaataagat aactgtggaa accctttcag ataaatacaa gctaagtagc 1440
   gaaggtacct tggaaatatc taacatacaa attgaagact caggaagata cacatgtgtt 1500
   gcccagaatg tccaaggggc agacactcgg gtggcaacaa ttaaggttaa cgggaccctt 1560
   ctggatggta cccaggtgct aaaaatatac gtcaagcaga cagaatccca ttccatctta 1620
   gtgtcctgga aagttaattc caatgtcatg acgtcaaact taaaatggtc gtctgccacc 1680
   atgaagattg ataaccctca cataacatat actgccaggg tcccagtcga tgtccatgaa 1740
   tacaacctaa cgcatctgca gccttccaca gattatgaag tgtgtctcac agtgtccaat 1800
   attcatcagc agactcaaaa gtcatgcgta aatgtcacaa ccaaaaatgc cgccttcgca 1860
   gtggacatct ctgatcaaga aaccagtaca gcccttgctg cagtaatggg gtctatgttt 1920
   gccgtcatta gccttgcgtc cattgctgtg tactttgcca aaagatttaa gagaaaaaac 1980
   taccaccact cattaaaaaa gtatatgcaa aaaacctctt caatcccact aaatgagctg 2040
   tacccaccac tcattaacct ctgggaaggt gacagcgaga aagacaaaga tggttctgca 2100
   gacaccaagc caacccaggt cgacacatcc agaagctatt acatgtggta actcagagga 2160
   tattttgctt ctggtagtaa ggagcacaaa gacgtttttg ctttattctg caaaagtgaa 2220
   caagttgaag acttttgtat ttttgacttt gctagtttgt ggcagagtgg agaggacggg 2280
   tggatatttc aaattttttt agtatagcgt atcgcaaggg tttgacacgg ctgccagcga 2340
   ctctaggctt ccagtctgtg tttggttttt attcttatca ttattatgat tgttattata 2400
   ttattatttt attttagttg ttgtgctaaa ctcaataatg ctgttctaac tacagtgctc 2460
   aataaaatga ttaatgacag gatggggttc ccctgtgctt ttaccagtag catgacccct 2520
   tctgaagcca tccgtagaaa gtactttgtc ctccaaaaag ctaacatatg gttttgaagc 2580
   agcattgaaa cttttgtagc aatctggtct atagactttt aactcaagaa gctaaggcta 2640
   gacttgttac cttcgttgaa tgatgttagt tgactgtact gtaatgttgt atcaactgaa 2700
   ttgaatgttt gcctttaaac aatgaatttt ctttttcttt cctttttttt ttttgttgta 2760
   atagttaaag aggcttagaa caagctaaca ggcaatagaa atatgtatat cagatttttt 2820
   aatgtaacaa actacatgtt aattgttatc ttattctttt tatctttagt agacactttt 2880
   aaaagaaaag acaattttgt tgtgtttaac tcaccaacac gtggtgtata atgaagacag 2940
   aactataata aattagtttt gttctgattt tttagaacac ttgcaataat gtatcattta 3000
   tagttcttgc tagttgcagt ggtaatattt ttcacatcca taaaaacaac taccaaaata 3060
<210> 2
   <211> 716
   <212> PRT
   <213> Homo sapiens
<220>
   <221> misc_feature
   <223> NLRR1
<400> 2
   Met Ala Arg Met Ser Phe Val Ile Ala Ala Cys Gln Leu Val Leu Gly
   1               5                   10                  15
   Leu Leu Met Thr Ser Leu Thr Glu Ser Ser Ile Gln Asn Ser Glu Cys
               20                  25                  30
   Pro Gln Leu Cys Val Cys Glu Ile Arg Pro Trp Phe Thr Pro Gln Ser
           35                  40                  45
   Thr Tyr Arg Glu Ala Thr Thr Val Asp Cys Asn Asp Leu Arg Leu Thr
       50                  55                  60
   Arg Ile Pro Ser Asn Leu Ser Ser Asp Thr Gln Val Leu Leu Leu Gln
   65                  70                  75                  80
   Ser Asn Asn Ile Ala Lys Thr Val Asp Glu Leu Gln Gln Leu Phe Asn
                   85                  90                  95
   Leu Thr Glu Leu Asp Phe Ser Gln Asn Asn Phe Thr Asn Ile Lys Glu
               100                 105                 110
   Val Gly Leu Ala Asn Leu Thr Gln Leu Thr Thr Leu His Leu Glu Glu
           115                 120                 125
   Asn Gln Ile Thr Glu Met Thr Asp Tyr Cys Leu Gln Asp Leu Ser Asn
       130                 135                 140
   Leu Gln Glu Leu Tyr Ile Asn His Asn Gln Ile Ser Thr Ile Ser Ala
   145                 150                 155                 160
   His Ala Phe Ala Gly Leu Lys Asn Leu Leu Arg Leu His Leu Asn Ser
                   165                 170                 175
   Asn Lys Leu Lys Val Ile Asp Ser Arg Trp Phe Asp Ser Thr Pro Asn
               180                 185                 190
   Leu Glu Ile Leu Met Ile Gly Glu Asn Pro Val Ile Gly Ile Leu Asp
           195                 200                 205
   Met Asn Phe Lys Pro Leu Ala Asn Leu Arg Ser Leu Val Leu Ala Gly
       210                 215                 220
   Met Tyr Leu Thr Asp Ile Pro Gly Asn Ala Leu Val Gly Leu Asp Ser
   225                 230                 235                 240
   Leu Glu Ser Leu Ser Phe Tyr Asp Asn Lys Leu Val Lys Val Pro Gln
                   245                 250                 255
   Leu Ala Leu Gln Lys Val Pro Asn Leu Lys Phe Leu Asp Leu Asn Lys
               260                 265                 270
   Asn Pro Ile His Lys Ile Gln Glu Gly Asp Phe Lys Asn Met Leu Arg
           275                 280                 285
   Leu Lys Glu Leu Gly Ile Asn Asn Met Gly Glu Leu Val Ser Val Asp
       290                 295                 300
   Arg Tyr Ala Leu Asp Asn Leu Pro Glu Leu Thr Lys Leu Glu Ala Thr
   305                 310                 315                 320
   Asn Asn Pro Lys Leu Ser Tyr Ile His Arg Leu Ala Phe Arg Ser Val
                   325                 330                 335
   Pro Ala Leu Glu Ser Leu Met Leu Asn Asn Asn Ala Leu Asn Ala Ile
               340                 345                 350
   Tyr Gln Lys Thr Val Glu Ser Leu Pro Asn Leu Arg Glu Ile Ser Ile
           355                 360                 365
   His Ser Asn Pro Leu Arg Cys Asp Cys Val Ile His Trp Ile Asn Ser
       370                 375                 380
   Asn Lys Thr Asn Ile Arg Phe Met Glu Pro Leu Ser Met Phe Cys Ala
   385                 390                 395                 400
   Met Pro Pro Glu Tyr Lys Gly His Gln Val Lys Glu Val Leu Ile Gln
                   405                 410                 415
   Asp Ser Ser Glu Gln Cys Leu Pro Met Ile Ser His Asp Ser Phe Pro
               420                 425                 430
   Asn Arg Leu Asn Val Asp Ile Gly Thr Thr Val Phe Leu Asp Cys Arg
           435                 440                 445
   Ala Met Ala Glu Pro Glu Pro Glu Ile Tyr Trp Val Thr Pro Ile Gly
       450                 455                 460
   Asn Lys Ile Thr Val Glu Thr Leu Ser Asp Lys Tyr Lys Leu Ser Ser
   465                 470                 475                 480
   Glu Gly Thr Leu Glu Ile Ser Asn Ile Gln Ile Glu Asp Ser Gly Arg
                   485                 490                 495
   Tyr Thr Cys Val Ala Gln Asn Val Gln Gly Ala Asp Thr Arg Val Ala
               500                 505                 510
   Thr Ile Lys Val Asn Gly Thr Leu Leu Asp Gly Thr Gln Val Leu Lys
           515                 520                 525
   Ile Tyr Val Lys Gln Thr Glu Ser His Ser Ile Leu Val Ser Trp Lys
       530                 535                 540
   Val Asn Ser Asn Val Met Thr Ser Asn Leu Lys Trp Ser Ser Ala Thr
   545                 550                 555                 560
   Met Lys Ile Asp Asn Pro His Ile Thr Tyr Thr Ala Arg Val Pro Val
                   565                 570                 575
   Asp Val His Glu Tyr Asn Leu Thr His Leu Gln Pro Ser Thr Asp Tyr
               580                 585                 590
   Glu Val Cys Leu Thr Val Ser Asn Ile His Gln Gln Thr Gln Lys Ser
           595                 600                 605
   Cys Val Asn Val Thr Thr Lys Asn Ala Ala Phe Ala Val Asp Ile Ser
       610                 615                 620
   Asp Gln Glu Thr Ser Thr Ala Leu Ala Ala Val Met Gly Ser Met Phe
   625                 630                 635                 640
   Ala Val Ile Ser Leu Ala Ser Ile Ala Val Tyr Phe Ala Lys Arg Phe
                   645                 650                 655
   Lys Arg Lys Asn Tyr His His Ser Leu Lys Lys Tyr Met Gln Lys Thr
               660                 665                 670
   Ser Ser Ile Pro Leu Asn Glu Leu Tyr Pro Pro Leu Ile Asn Leu Trp
           675                 680                 685
   Glu Gly Asp Ser Glu Lys Asp Lys Asp Gly Ser Ala Asp Thr Lys Pro
       690                 695                 700
   Thr Gln Val Asp Thr Ser Arg Ser Tyr Tyr Met Trp
   705                 710                 715
<210> 3
   <211> 21
   <212> RNA
   <213> Homo sapiens
<220>
   <221> misc_feature
   <223> siRNA for NLRR1
<400> 3
   ucuugguuga gcuguguagu u 21
<210> 4
   <211> 21
   <212> RNA
   <213> Homo sapiens
<220>
   <221> misc_feature
   <223> siRNA for NLRR1
<400> 4
   aacuacacag cucaaccaag a 21
<210> 5
   <211> 20
   <212> DNA
   <213> Homo sapiens
<220>
   <221> misc_feature
   <223> NLRR1 forward primer
<400> 5
   aattcgtccc tggtttaccc 20
<210> 6
   <211> 20
   <212> DNA
   <213> Homo sapiens
<220>
   <221> misc_feature
   <223> NLRR1reverse primer
<400> 6
   agcccgacct ccttaatgtt 20
<210> 7
   <211> 20
   <212> DNA
   <213> Homo sapiens
<220>
   <221> misc_feature
   <223> GAPDH forward primer
<400> 7
   acctgacctg ccgtctagaa 20
<210> 8
   <211> 20
   <212> DNA
   <213> Homo sapiens
<220>
   <221> misc_feature
   <223> GAPDH reverse primer
<400> 8
   tccaccaccc tgttgctgta 20